# EUROPEAN PATENT SPECIFICATION

(11) **EP 3 518 660 B1**
(45) Date of publication and mention of the grant of the patent: **09.12.2026**
(21) Application number: 17783426.4
(22) Date of filing: 29.09.2017
(51) Int. Cl.: A01H 5/12, C07K 14/415, C12N 15/82

(54) **PERONOSPORA RESISTANCE IN SPINACIA OLERACEA**
PERONOSPORA-RESISTENZ BEI SPINACIA OLERACEA
RÉSISTANCE AU PARASITE PERONOSPORA DANS SPINACIA OLERACEA

(30) Priority: 30.09.2016 WO PCT/EP2016/073505
(43) Date of publication of application: 07.08.2019
(73) Proprietor: Rijk Zwaan Zaadteelt en Zaadhandel B.V., 2678 KX De Lier (NL)
(72) Inventor: KOCK, Vincent Laurens Adrianus, 2678 KX De Lier (NL); FEITSMA, Johannes Geert Jan, 2678 KX De Lier (NL); FRIJTERS, Raoul Jacobus Johannes Maria, 2678 KX De Lier (NL)
(74) Representative: Arnold & Siedsma
(86) International application number: PCT/EP2017/074810
(87) International publication number: WO 2018/060445

(56) References cited:
- WO-A1-2015/036469
- DE-A1- 102013 010 026
- US-A1- 2005 183 150
- US-B1- 9 402 363
- US-B2- 9 265 275
- CORRELL J C ET AL: "Spinach: better management of downy mildew and white rust through genomics", EUROPEAN JOURNAL OF PLANT PATHOLOGY, KLUWER ACADEMIC PUBLISHERS, DO, vol. 129, no. 2, 4 December 2010 (2010-12-04), pages 193 - 205, XP019872925, ISSN: 1573-8469, DOI: 10.1007/S10658-010-9713-Y

## Description

### FIELD OF THE INVENTION

The invention relates to an allele capable of conferring resistance to a spinach plant against multiple *Peronospora farinosa* f. sp. *spinaciae* races. The invention also relates to a spinach plant, to propagation material of said spinach plant, and to a cell of said spinach plant. It also relates to methods for identifying and selecting such plants.

### BACKGROUND OF THE INVENTION

Downy mildew (*Peronospora farinosa* f. sp. *spinaciae*) is a major threat for spinach growers because it directly affects the harvested leaves. In spinach, downy mildew is caused by the oomycete *Peronospora farinosa* f. sp. *spinaciae* (formerly known as *P. effusa*)*.* Infection makes the leaves unsuitable for sale and consumption, as it manifests itself phenotypically as yellow lesions on the older leaves, and on the abaxial leaf surface a greyish fungal growth can be observed. The infection can spread very rapidly, and it can occur both in glasshouse cultivation and in soil cultivation. The optimal temperature for formation and germination of *P. farinosa* f. sp. *spinaciae* spores is 9 to 12°C, and it is facilitated by a high relative humidity. When spores are deposited on a humid leaf surface they can readily germinate and infect the leaf. Fungal growth is optimal between 8 and 20°C and a relative humidity of ≥80%, and within 6 and 13 days after infection mycelium growth can be observed. Oospores of *P. farinosa* can survive in the soil for up to 3 years, or as mycelium in seeds or living plants.

To date 16 pathogenic races of spinach downy mildew (Pfs) have been officially identified and characterized, and many new candidates are observed in the field. The 16 officially recognised races of *Peronospora farinosa* f. sp. *spinaciae,* are designated Pfs:1 to Pfs:16 (Irish et al. Phtypathol. Vol. 98 pg. 894-900, 2008; Plantum NL (Dutch association for breeding, tissue culture, production and trade of seed and young plants) press release, "Benoeming van Pfs: 14, een nieuwe fysio van valse meeldauw in spinazie", September 19, 2012; Report Jim Correl (Univ. Arkansas) and Steven Koike (UC Cooperative Extension, Monterey County), "Race Pfs: 14 - Another new race of the spinach downy mildew pathogen", September 18, 2012; Plantum NL press release, "Denomination of Pfs: 15, a new race of downy mildew in spinach", September 2, 2014, Plantum NL press release, "Denomination of Pfs: 16, a new race of downy mildew in spinach, March 15, 2016). Races 4 to 15 were identified between 1990 and 2014, while only recently another new *Peronospora* isolate has been identified, termed UA201519B, which subsequently has been officially named Pfs:16 by the International Working Group on Peronospora (IWGP) (Plantum NL (Dutch association for breeding, tissue culture, production and trade of seed and young plants) press release, "Denomination of Pfs: 16, a new race of downy mildew in spinach", March 15, 2016. All 16 officially recognized Pfs races are publicly available from the Department of Plant Pathology, University of Arkansas, Fayetteville, AR 72701, USA, and also from NAK Tuinbouw, Sotaweg 22, 2371 GD Roelofarendsveen, the Netherlands.

Especially the latest identified *Peronospora* races can break the resistance of many spinach varieties that are currently used commercially worldwide, and they thus pose a serious threat to the productivity of the spinach industry. Therefore, it is crucial to stay at the forefront of developments in this field, as *Peronospora* continuously develops the ability to break the resistances that are present in commercial spinach varieties. For this reason new resistance genes against downy mildew are very valuable assets, and they form an important research focus in breeding and particular in spinach and lettuce breeding. One of the main goals of spinach breeders is to rapidly develop spinach varieties with a resistance to as many *Peronospora* races as possible, including the latest identified races, before these races become wide-spread and pose a threat to the industry.

In commercial spinach varieties resistance against downy mildew is usually caused by so-called R-genes. R-gene mediated resistance is based on the ability of a plant to recognize the invading pathogen. In many cases this recognition occurs after the pathogen has established the first phases of interaction and transferred a so called pathogenicity (or avirulence) factor into the plant cell. These pathogenicity factors interact with host components in order to establish conditions which are favorable for the pathogen to invade the host and thereby cause disease. When a plant is able to recognize the events triggered by the pathogenicity factors a resistance response can be initiated. In many different plant pathogen interaction systems such as the interaction of spinach with different downy mildew strains, the plant initiates these events only after specific recognition of the invading pathogen.

Co-evolution of plant and pathogen has led to an arms race in which a R-gene mediated resistance is sometimes overcome as a consequence of the capability of the pathogen to interact with and modify alternative host targets or the same targets in a different way, such that the recognition is lost and infection can be established successfully resulting in disease. In order to re-establish resistance in a plant, a new R-gene has to be introduced which is able to recognize the mode of action of an alternative pathogenicity factor.

Despite the fact that the durability of R-genes is relatively low, R-genes are in spinach still the predominant form of defense against downy mildew. This is mainly due to the fact that it is the only form of defense that gives absolute resistance. So far plant breeders have been very successful in generating downy mildew resistant spinach varieties by making use of resistance genes residing in the wild germplasm of the crop species. Even though R-genes are extensively used in spinach breeding, until now not much is known of these R-genes. The R-genes present in the current commercial spinach varieties have never been characterized at the molecular level, i.e. their sequence until now was unknown. Also up until now there are no closely linked molecular markers known in the art that separate these R-genes, nor are the molecular characteristics of the genes themselves known in the art. Therefore, the search for new R-genes and R-gene identification is currently based on phenotypic assays in which many accessions are screened for possible variation in their resistance pattern. Subsequently it has to be determined through crossing and selection whether a newly observed resistance is in fact caused by an R-gene.

Adequately responding to newly emerging downy mildew races is crucial for developing commercially successful spinach varieties. Therefore, it is the object of the invention to provide a new resistance allele conferring resistance to a newly emerged downy mildew isolate and to provide molecular biological tools for identifying this new resistance allele.

### SUMMARY OF THE INVENTION

In the research leading to the present invention, it was found that different resistance genes that confer resistance to *Peronospora farinosa* f. sp. *spinaciae* in spinach are not separate resistance loci, as had been previously assumed, but that they are different alleles of the same one or two genes. These one or two genes, which are either *"alpha-WOLF"* type or *"beta-WOLF"* type genes (together referred to as "the *WOLF* genes") each encode a protein that belongs to the CC-NBS-LRR family (Coiled Coil - Nucleotide Binding Site - Leucine-Rich Repeat). Depending on the allelic variant (or the allelic variants) that is (are) present in a spinach plant, said plant will produce a variant of the WOLF protein that confers a certain resistance profile to pathogenic races of *Peronospora farinosa* f. sp. *spinaciae.* The research leading to the present invention has furthermore elucidated the relationship between the different alleles present in the genome of a spinach plant and the resistance profile of said plant to a number of different pathogenic races of *Peronospora farinosa* f. sp. *spinaciae.*

In the context of this invention the term "allele" or "allelic variant" is used to designate a version of the gene that is linked to a specific phenotype, i.e. resistance profile. It was found that a spinach plant may carry one or two *WOLF* genes. Each of these two *WOLF* genes encompasses multiple alleles, each allele conferring a particular resistance profile. The beta *WOLF* gene is located on scaffold12735 (sequence: GenBank: KQ143339.1), at position 213573-221884. In case the spinach plant also carries or only carries the alpha-WOLF gene, the alpha-WOLF gene is located at approximately the same location as where the beta-WOLF gene is located on scaffold12735 in the Viroflay genome assembly.

A screen for novel *WOLF*-alleles in the spinach germplasm identified a new allele of the alpha-*WOLF* gene conferring a new and unique resistance profile against several downy mildew races including the recently identified race pfs:16.

### DETAILED DESCRIPTION OF THE INVENTION

A genome assembly for spinach variety Viroflay - which is susceptible to all known pathogenic races of *Peronospora farinosa* f. sp. *spinaciae* - is publicly available (*Spinacia oleracea* cultivar SynViroflay, whole genome shotgun sequencing project; Bioproject: PRJNA41497; GenBank: AYZV00000000.2; BioSample: SAMN02182572, see also Dohm et al, 2014, Nature 505: 546-549). In this genome assembly for Viroflay, the beta*-WOLF* gene is located on scaffold12735 (sequence: GenBank: KQ143339.1), at position 213573-221884. The sequence covered by this interval comprises the entire genomic sequence of the beta-*WOLF* gene of Viroflay, plus 2000 basepairs sequence upstream from the gene, plus the sequence downstream from the gene, up to the locus of the neighbouring gene that is situated downstream from the *WOLF* gene. Spinach variety Viroflay only possesses a single *WOLF* gene, namely a beta-*WOLF* gene, but most other spinach lines harbor a single alpha-type *WOLF* gene at the same location in the genome. Other spinach lines harbor two *WOLF* genes at approximately the same location in the genome. In such cases, the two *WOLF* genes are positioned adjacent to each other. In most spinach lines that harbor two *WOLF* genes, one of said *WOLF* genes belongs to the alpha-type, and the other *WOLF* gene belongs to the beta-type. In the research leading to the present invention, it was observed that this allelic variation in the *WOLF* locus is responsible for differences in resistance to pathogenic races of *Peronospora farinosa* f. sp. *spinaciae.*

The difference between an allele of an alpha*-WOLF* gene and an allele of a beta-*WOLF* gene lies in the presence of specific conserved amino acid motifs in the encoded protein sequence. As mentioned above, all WOLF proteins possess - from N- to C-terminus - the following domains that are generally known in the art: a coiled coil domain (RX-CC-like, cd14798), an NBS domain (also referred to as "NB-ARC domain", pfam00931; van der Biezen & Jones, 1998, *Curr. Biol.* 8: R226-R228), and leucine-rich repeats (IPR032675) which encompass the LRR domain. In addition, all WOLF proteins comprise in their amino acid sequence the motif "MAEIGYSVC" at the N-terminus. In addition to this, all alpha-WOLF proteins comprise the motif "KWMCLR" in their amino acid sequence, whereas all beta-WOLF proteins comprise the motif "HVGCVVDR" in their amino acid sequence.

The present invention relates to a new *Peronospora farinosa* f. sp. *spinaciae* resistance conferring allele of the alpha-WOLF gene designated alpha-WOLF 8.

In particular, the invention relates to an allele designated alpha-WOLF 8 which confers resistance to at least one *Peronospora farinosa* f. sp. *spinaciae* race when present in a spinach plant, wherein the allele has a genomic nucleotide sequence of SEQ ID No:1, a coding sequence of SEQ ID No:2, SEQ ID No:3 or SEQ ID No:4, said allele encoding a CC-NBS-LRR protein that comprises in its amino acid sequence: a) the motif "MAEIGYSVC" at its N-terminus; and b) the motif "KWMCLR"; wherein the allele when homozygously present in a spinach plant confers complete resistance to *Peronospora farinosa* f. sp. *spinaciae* races pfs:1, pfs:2, pfs:6, pfs:8 and pfs:15, and confers intermediate resistance to pfs:5, pfs:10 and pfs:16, and does not confer resistance to pfs:3, pfs:4, pfs:7, pfs:9, pfs:11, pfs:12, pfs:13 and pfs:14. An alpha WOLF 8 allele can further comprise an additional motif in their amino acid sequence, namely "DQEDEGEDN".

For the purpose of this invention, the LRR domain of the protein of the alpha-WOLF 8 allele is defined as the amino acid sequence that in order of increased preference has at least 92%, 93%, 94%, 95%, 96%, 97%, 98%, 99%, 100% sequence similarity to SEQ ID No:12.

The skilled person is familiar with methods for the calculation of sequence similarity. Suitably sequence similarity is calculated using EMBOSS stretcher 6.6.0 (www.ebi.ac.uk/Tools/psa/emboss stretcher), using the EBLOSUM62 matrix and the resulting "similarity score".

The LRR domain of the alpha-WOLF 8 allele as defined herein can be determined by amplifying and sequencing the genomic DNA encoding for the amino acid sequence of LRR domain using specific primers, and subsequently translating the DNA sequence into an amino acid sequence, thereby applying common sense in choosing the correct reading frame. The skilled person is capable of doing this, using freely available online bioinformatics tools such as can be found here: http://web.expasy.org/translate/

The genomic sequence of an LRR domain of an alpha-WOLF gene such as alpha-WOLF 8 can be amplified using a primer pair having a forward primer which is a nucleic acid molecule having the sequence of SEQ ID No:8 and a reverse primer which is a nucleic acid molecule having the sequence of SEQ ID No:9.

PCR conditions for amplifying the LRR domain-encoding region of an alpha- *WOLF* gene using primers having SEQ ID No:8 and SEQ ID No:9 are, using Platinum Taq enzyme (Thermo Fisher Scientific): 3 minutes at 95°C (initial denaturing step); 40 amplification cycles, each cycle consisting of: 30 seconds denaturation at 95°C, 30 seconds annealing at 60°C, and 30 seconds extension at 72°C; 2 minutes at 72°C (final extension step).

The LRR domain of a beta-WOLF gene, e.g. the null allele as present in variety Viroflay, can be amplified using a forward primer which is a nucleic acid molecule having the sequence of SEQ ID No:10 and a reverse primer which is a nucleic acid molecule having the sequence of SEQ ID No:9.

PCR conditions for amplifying the LRR domain-encoding region of a beta- *WOLF* gene using primers having SEQ ID No:9 and SEQ ID No:10 are as follows, using Platinum Taq enzyme (Thermo Fisher Scientific):- 3 minutes at 95°C (initial denaturing step); 40 amplification cycles, each cycle consisting of: 30 seconds denaturation at 95°C, 50 seconds annealing at 58°C and 50 seconds extension at 72°C; 2 minutes at 72°C (final extension step).

Therefore, the invention also relates to a primer pair for amplifying the LRR domain of an alpha-WOLF gene, more in particular for amplifying the LRR domain of an alpha-WOLF 8 allele wherein the forward primer is a nucleic acid molecule having the sequence of SEQ ID No:8 and the reverse primer which is a nucleic acid molecule having the sequence of SEQ ID No:9. The primers disclosed herein have been specifically designed for selectively amplifying part of a *WOLF* gene, and not of any other CC-NBS-LRR protein-encoding genes.

Disclosed herein is an alpha-WOLF 8 allele which has a genomic sequence that in order of increased preference has at least 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:1.

Further disclosed herein is an alpha-WOLF 8 allele which has a coding sequence which in order of increased preference has at least 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:2. This is the first splice variant of the alpha-WOLF 8 allele.

Also disclosed herein is an alpha-WOLF 8 allele which has a coding sequence which in order of increased preference has at least 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:3. This is the second splice variant.

Additionally, disclosed herein is an alpha-WOLF 8 allele which has a coding sequence which in order of increased preference has at least 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:4. This is the third splice variant.

Also disclosed herein is an alpha-WOLF 8 allele which encodes for a protein having an amino acid sequence which in order of increased preference has at least 92%, 93%, 94%, 95%, 96%, 97%, 98%, 99%, 100% sequence similarity to SEQ ID No:5.

Furthermore, disclosed herein is an alpha-WOLF 8 allele which encodes for a protein having an amino acid sequence which in order of increased preference has at least 92%, 93%, 94%, 95%, 96%, 97%, 98%, 99%, 100% sequence similarity to SEQ ID No:6.

In addition, disclosed herein is an alpha-WOLF 8 allele which encodes for a protein having an amino acid sequence which in order of increased preference has at least 92%, 93%, 94%, 95%, 96%, 97%, 98%, 99%, 100% sequence similarity to SEQ ID No:7.

The alpha-WOLF 8 allele when homozygously present in a spinach plant confers complete resistance to the officially recognized *Peronospora farinosa* f. Sp. *spinacea* races pfs:1, pfs:2, pfs:6, pfs:8 and pfs:15, and confers intermediate resistance to pfs:5, pfs:10 and pfs:16, and does not confer resistance to downy mildew races pfs:3, pfs:4, pfs:7, pfs:9, pfs:11, pfs:12, pfs:13 and pfs:14 (See **Table 1).** As indicated in **Table 1,** a spinach plant heterozygous for the alpha-WOLF 8 allele and not carrying any other resistance conferring allele will be susceptible for downy mildew races Pfs:5, Pfs:10, and Pfs:16.

The resistance of a spinach plant against one or more races of *Peronospora farinosa f.* sp. *Spinaciae* can be determined using a seedling test. Herein, a seedling test is defined as a test wherein spinach plants are planted in trays containing growth medium, optionally fertilized twice a week after seedling emergence. Plants were inoculated at the first true leaf stage with a sporangial suspension having a concentration of approximately 2.5 × 10⁵/ml of one of the pathogenic races of *Peronospora farinosa* f. sp. *spinaciae* or isolates to be tested. The inoculated plants are placed in a dew chamber at 18°C with 100% relative humidity for a 24 h period, and then moved to a growth chamber at 18°C with a 12 h photoperiod for 6 days. After 6 days, the plants are returned to the dew chamber for 24 h to induce sporulation, and subsequently scored for a disease reaction. Preferably, 30 plants per race are tested.

As used herein, a plant is completely resistant against a *Peronospora farinosa* f. sp. *spinaciae* race when a plant shows no symptoms in the seedling test described herein.

As used herein, a plant is intermediately resistant against a *Peronospora farinosa f.* sp. *spinaciae* race when a plant shows only symptoms of chlorosis, or sporulation occurring only on the tips of the cotyledons in the seedling test described herein.

As used herein, a plant is susceptible to an isolate of a *Peronospora farinosa* f. sp. *spinaciae* race when a plant shows more than only symptoms of chlorosis, or when sporulation occurs on area larger than only the tips of the cotyledons in the seedling test described herein.

Another aspect of the invention relates to a spinach plant, comprising the alpha-WOLF 8 allele of invention, of which a representative sample of seed was deposited with the NCIMB under NCIMB accession number 42646.

A plant which comprises an alpha-WOLF 8 allele may be an agronomically elite spinach plant. An agronomically elite spinach plant is a plant having a genotype that results into an accumulation of distinguishable and desirable agronomic traits which allow a producer to harvest a product of commercial significance, preferably an agronomically elite spinach plant comprising an alpha-WOLF 8 allele is a plant of an inbred line or a hybrid.

As used herein, a plant of an inbred line is a plant of a population of plants that is the result of three or more rounds of selfing, or backcrossing; or which plant is a double haploid. An inbred line may e.g. be a parent line used for the production of a commercial hybrid.

As used herein, a hybrid plant is a plant which is the result of a cross between two different plants having different genotypes. More in particular, a hybrid plant is the result of a cross between plants of two different inbred lines, such a hybrid plant may e.g. be a plant of an F₁ hybrid variety.

A plant carrying an alpha-WOLF 8 allele in heterozygous form may further comprise a beta-WOLF 0 allele as e.g. present in variety Viroflay wherein the beta-WOLF 0 allele does not confer any resistance to downy mildew. However, a plant heterozygous for the alpha-WOLF 8 allele may further comprise an allele of the alpha/beta-WOLF gene that does provide resistance to downy mildew. Preferably, such an allele would complement the alpha-WOLF 8 allele such that the spinach plant will be at least intermediately resistant to one or more other races to which the alpha-WOLF 8 allele does not provide resistance. Most preferably the other allele of the alpha/beta-*WOLF* gene complements the alpha-WOLF 8 allele such that the plant is resistant to *Peronospora farinosa* f. sp. *spinaciae* races Pfs:1 to Pfs:16. In one embodiment such a plant is an agronomically elite plant.

Alternatively, the resistance profile of a plant carrying the alpha-WOLF 8 allele is complemented by a resistance conferring allele of a totally different gene. Examples of such genes are e.g. DMR1 as described in US8,354,570 and DMR6 as described in US9,121,029.

The invention thus relates to a spinach plant carrying the alpha-WOLF 8 allele and further comprising a genetic determinant resulting in resistance against *Peronospora farinosa* f. Sp. *spinacea* races pfs:1 to pfs:16. The genetic determinant can be another resistance conferring alpha/beta-WOLF allele or a resistance conferring allele of a totally different gene.

The invention further relates to propagation material comprising the alpha-WOLF 8 allele. In one embodiment, the propagation material is suitable for sexual reproduction. Such propagation material comprises for example a microspore, pollen, ovary, ovule, embryo sac and egg cell. In another embodiment, the propagation material is suitable for vegetative reproduction. Such propagation material comprises for example a cutting, root, stem, cell, protoplast, and a tissue culture of regenerable cells. A part of the plant that is suitable for preparing tissue cultures is in particular a leaf, pollen, an embryo, a cotyledon, a hypocotyl, a meristematic cell, a root tip, an anther, a flower, a seed and a stem.

The invention furthermore relates to a cell of a spinach plant comprising the alpha-WOLF 8 allele. Such a cell may be either in isolated form or may be part of the complete plant or parts thereof and then still constitutes a cell of the invention because such a cell harbors the alpha-WOLF 8 allele that confers resistance to downy mildew. Each cell of a plant of the invention carries the genetic information that confers resistance to *Peronospora farinosa* f. sp. *spinaciae .* Such a cell of the invention may also be a regenerable cell that can be used to regenerate a new plant comprising the allele of the invention.

Disclosed herein is a method for making a hybrid spinach seed comprising crossing a first parent spinach plant with a second parent spinach plant and harvesting the resultant hybrid spinach seed, wherein said first and/or second parent spinach plant comprises the alpha-WOLF 8 allele. In particular the first and/or second parent plant is a plant of an inbred line as defined herein.

The invention further relates hybrid spinach plant grown from seed produced by crossing a first parent spinach plant with a second parent spinach plant and harvesting the resultant hybrid spinach seed, wherein said first and/or second parent spinach plant comprises the alpha-WOLF 8 allele.

Another aspect of the invention relates to a method for identifying or selecting a spinach plant carrying the alpha-WOLF 8 allele, comprising determining the presence of a genomic nucleotide sequence or a part thereof in the genome of a plant, wherein said sequence has the sequence of SEQ ID No:1. Disclosed herein is a method for identifying or selecting a spinach plant carrying the alpha-WOLF 8 allele, comprising determining the presence of a genomic nucleotide sequence or a part thereof in the genome of a plant, wherein said sequence has in order of increased preference 92%, 93%, 94%, 95%, 96%, 97%, 98%, 99%, 100% sequence similarity to SEQ ID No:1.

The invention further relates a method for identifying or selecting a spinach plant carrying the alpha-WOLF 8 allele, comprising determining the presence of a coding sequence or a part thereof in the genome of a plant, wherein said sequence has the sequence of SEQ ID No:2. Disclosed herein is a method for identifying or selecting a spinach plant carrying the alpha-WOLF 8 allele, comprising determining the presence of a coding sequence or a part thereof in the genome of a plant, wherein said sequence has in order of increased preference 92%, 93%, 94%, 95%, 96%, 97%, 98%, 99%, 100% sequence similarity to SEQ ID No:2.

The invention further relates a method for identifying or selecting a spinach plant carrying the alpha-WOLF 8 allele, comprising determining the presence of a coding sequence or a part thereof in the genome of a plant, wherein said sequence has the sequence of SEQ ID No:3. Disclosed herein is a method for identifying or selecting a spinach plant carrying the alpha-WOLF 8 allele, comprising determining the presence of a coding sequence or a part thereof in the genome of a plant, wherein said sequence has in order of increased preference 92%, 93%, 94%, 95%, 96%, 97%, 98%, 99%, 100% sequence similarity to SEQ ID No:3.

The invention further relates a method for identifying or selecting a spinach plant carrying the alpha-WOLF 8 allele, comprising determining the presence of a coding sequence or a part thereof in the genome of a plant, wherein said sequence has the sequence of SEQ ID No:4. Disclosed herein is a method for identifying or selecting a spinach plant carrying the alpha-WOLF 8 allele, comprising determining the presence of a coding sequence or a part thereof in the genome of a plant, wherein said sequence has in order of increased preference 92%, 93%, 94%, 95%, 96%, 97%, 98%, 99%, 100% sequence similarity to SEQ ID No:4.

Determining the genomic DNA or coding DNA sequence of at least part of a *WOLF* gene in the genome of a spinach plant may be performed using any suitable molecular biological method known in the art, including but not limited to (genomic) PCR amplification followed by Sanger sequencing, whole-genome-sequencing, transcriptome sequencing, sequence-specific target capture followed by next-generation sequencing (using, for example, the xGen^{®} target capture system of Integrated DNA Technologies), specific amplification of LRR-domain-comprising gene sequences (using, for example, the RenSeq methodology, as described in US patent application 14/627116, and in Jupe et al., 2013, Plant J. 76: 530-544) followed by sequencing, etcetera.

In another embodiment the invention relates to a method for identifying or selecting a plant carrying the alpha-WOLF 8 allele which comprises determining the DNA sequence coding for the LRR domain, wherein said LRR domain has the sequence of SEQ ID No. 11..

In a further embodiment of the method the LRR domain of the alpha-WOLF 8 allele is determined by using a primer pair to amplify the genomic DNA region of the LRR domain. The forward primer is preferably a nucleic acid molecule having the sequence of SEQ ID No:8 and the reverse primer is preferably a nucleic acid molecule having the sequence of SEQ ID No:9.

Disclosed herein is a method for producing a spinach plant comprising resistance to *Peronospora farinosa* f. sp. *spinaciae* comprising: (a) crossing a plant comprising the alpha-WOLF 8 allele, with another plant; (b) optionally performing one or more rounds of selfing and/or crossing; (c) optionally selecting after each round of selfing or crossing for a plant that comprises the alpha-WOLF 8 allele.

Selecting a plant comprising an alpha-WOLF 8 allele can be done genotypically by determining the presence of the genomic DNA sequence of the allele having in order of increased preference 80%, 81%, 82%, 83%, 84%,85%, 86%, 87%, 88%, 89%, 90%, 91%, 92%, 93%, 94%, 95%, 96%, 97%, 98%, 99%, 100%sequence similarity to SEQ ID No:1.

Selecting a plant comprising an alpha-WOLF 8 allele can be done genotypically by determining the presence the coding sequence of the allele having in order of increased preference 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:2,

Selecting a plant comprising an alpha-WOLF 8 allele can be done genotypically by determining the presence the coding sequence of the allele having in order of increased preference 80%, 81%, 82%, 83%, 84%,85%, 86%, 87%, 88%, 89%, 90%, 91%, 92%, 93%, 94%, 95%, 96%, 97%, 98%, 99%, 100%sequence similarity to SEQ ID No:3.

Selecting a plant comprising an alpha-WOLF 8 allele can be done genotypically by determining the presence the coding sequence of the allele having in order of increased preference 80%, 81%, 82%, 83%, 84%,85%, 86%, 87%, 88%, 89%, 90%, 91%, 92%, 93%, 94%, 95%, 96%, 97%, 98%, 99%, 100%sequence similarity to SEQ ID No:4.

Alternatively, the presence of an alpha-WOLF 8 allele can be determined phenotypically by assaying a plant in a disease test, for example the test as described herein, and identifying a plant carrying the alpha-WOLF 8 allele based on the resistance pattern as described herein and indicated in **Table 1.**

Disclosed herein is the use of a spinach plant carrying the alpha-WOLF 8 allele in breeding to confer resistance against *Peronospora farinosa* f. sp. *spinaciae.*

Also disclosed herein is a breeding method for the development of spinach plants carrying the alpha-WOLF 8 allele of the invention wherein germplasm which comprises said allele is used. Seed capable of growing into a plant comprising the allele of the invention and being representative for the germplasm was deposited with the NCIMB under deposit number NCIMB 42646.

Disclosed herein is a method for the production of a spinach plant which comprises the alpha-WOLF 8 allele, which method comprises: (a) crossing a plant comprising the allele with another plant; (b) optionally selecting for plants comprising said allele in the F1; (c) optionally backcrossing the resulting F1 with the preferred parent and selecting for plants that have the said allele in the BC1F1; (d) optionally performing one or more additional rounds of selfing, crossing, and/or backcrossing, and subsequently selecting for a plant which comprises the said allele or shows the resistance profile corresponding to said allele. Also disclosed herein is a spinach plant produced by this method.

Further disclosed herein is a harvested leaf of a spinach plant of the invention, to a food product which comprises a harvested leaf of a spinach plant of the invention, either in natural or in processed form.

Spinach leaves are sold in packaged form, including without limitation as prepackaged spinach leaves or as processed in a salad comprising said leaves. Mention of such a package is e.g. made in US Patent No. 5,523,136, which provides packaging film, and packages from such packaging film, including such packaging containing leafy produce, and methods for making and using such packaging film and packages, which are suitable for use with the spinach leaves. Thus, disclosed herein are the use of and methods for making and using the leaves of the spinach plant of the invention, as well as leaves of spinach plants derived from the invention.

Also disclosed herein is a container which comprises one or more plants of the invention, or one or more spinach plants derived from a plant of the invention, in a growth substrate for harvest of leaves from the plant, in a domestic environment. This way the consumer may pick very fresh leaves for use in salads, when the plant is in a ready-to-harvest condition.

Disclosed herein is the use of a spinach plant, of which representative seed was deposited with the NCIMB under accession number NCIMB 42646, in the production of a spinach plant comprising the alpha-WOLF 8 allele.

A spinach plant may be a hybrid, doubled haploid, or inbred spinach plant.

Disclosed herein is the use of a cell comprising the alpha-WOLF 8 allele for the production of a spinach plant showing resistance to *Peronospora farinosa* f. sp. *spinaciae.*

Also disclosed herein is the use of a tissue culture comprising the alpha-WOLF 8 allele for the production of a spinach plant showing resistance to *Peronospora farinosa* f. sp. *spinaciae.*

### RESISTANCE INFORMATION

**Table 1.**

| Resistance profile conferred by the Alpha-WOLF 8 allele. A "-" means complete resistance against a particular downy mildew race; "(-)" means intermediate resistance against a particular downy mildew race; "+" means that the allele confers no resistance and would cause a plant only carrying the Alpha*-WOLF* 8 allele to be fully susceptible for that particular downy mildew race. | | | |
|---|---|---|---|
| *The intermediate resistances against Pfs:5, Pfs:10, and Pfs:16 conferred by the alpha-WOLF 8 allele are only observed in homozygous state. A plant carrying the allele in heterozygous state and not carrying any other resistance conferring allele (i.e. carrying the beta-WOLF zero allele) would be susceptible for Pfs:5, Pfs:10, and Pfs:16. | | | |
| **Alpha-WOLF 8 resistance profile** | | | |
| ***Peronospora farinosa* f*.* sp. *Spinaciae* race** | **Resistance score** | ***Peronospora farinosa* f. sp. *Spinaciae* race** | **Resistance score** |
| Pfs:1 | - | Pfs:9 | + |
| Pfs:2 | - | Pfs:10 | (-)* |
| Pfs:3 | + | Pfs:11 | + |
| Pfs:4 | + | Pfs:12 | + |
| Pfs:5 | (-)* | Pfs:13 | + |
| Pfs:6 | - | Pfs:14 | + |
| Pfs:7 | + | Pfs:15 | - |
| Pfs:8 | - | Pfs:16 | (-)* |

### DEPOSIT INFORMATION

Seeds of a plant 16R.58468 that comprises the alpha-WOLF 8 allele of the invention in its genome were deposited with NCIMB Ltd, Ferguson Building, Craibstone Estate, Bucksburn, Aberdeen AB21 9YA, UK, on September 9 2016 , under deposit accession number 42646. The deposit was made pursuant to the terms of the Budapest Treaty. Upon issuance of a patent, all restrictions upon the deposit will be removed, and the deposit is intended to meet the requirements of 37 CFR § 1.801-1.809. The deposit will be irrevocably and without restriction or condition released to the public upon the issuance of a patent. The deposit will be maintained in the depository for a period of 30 years, or 5 years after the last request, or for the effective life of the patent, whichever is longer, and will be replaced if necessary during that period.

### SEQUENCE INFORMATION

**Table 2. Sequence information.**

| | |
|---|---|
| SEQ ID No:1: Genomic sequence of alpha-WOLF 8 | ATGGCCGAAATCGGATACTCGGTTTGTGCGAAACTCATCGAAGTG ATTGGCAGTGAGCTGATCAAAGAGATTTGTGACACATGGGGTTAC AAATCTCTTCTTGAGGACCTCAACAAAACTGTATTGACGGTCAGG AACGTTCTCATTCAAGCCGGGGTGATGCGGGAGCTTACTAGTGAA CAACAAGGTTTCATTGCAGACCTTAAAGATGTTGTTTATGATGCTG ATGACTTGTTCGACAAGTTACTCACTCGTGCTGAGCGAAAACAGA TTGATGGAAACGAAATCTCTGAAAAGGTACGTCGTTTCTTTTCCTC TAGTAACAAGATCGGTCAAGCTTACTACATGTCTCGTAAGGTTAA GGAAATTAAGAAGCAGTTGGATGAAATTGTTGATAGGCATACAAA ATTTGGGTTTAGTGCCGAGTTTATACCTGTTTGTAGGGAAAGGGGG AACGAGAGGGAAACACGTTCATATATAGATGTCAAGAATATTCTT GGGAGGGATAAAGATAAGAATGATATCATAGATAGGTTGCTTAAT CGTAATGGTAATGAAGCTTGTAGTTTCCTGACCATAGTGGGAGCG GGAGGATTGGGAAAAACTGCTCTTGCACAACTTGTGTTCAATGAT GAAAGGGTCAAAATTGAGTTCCATGATTTGAGGTATTGGGTTTGT GTCTCTGATCAAGATGGGGGCCAATTTGATGTGAAAGAAATCCTT TGTAAGATTTTAGAGGTGGTTACTAAGGAGAAAGTTGATAATAGT TCCACATTGGAATTGGTACAAAGCCAATTTCAAGAGAAGTTAAGA GGAAAGAAGTACTTCCTTGTTCTTGATGATGTATGGAACGAAGAT CGTGAGAAGTGGCTTCCTTTGGAAGAGTTGTTAATGTTGGGTCAA GGGGGAAGCAAGGTTGTAGTGACCGCACGTTCAGAGAAGACAGC AAATGTCATAGGGAAAAGACATTTTTATACACTGGAATGTTTGTC ACCAGATTATTCATGGAGCTTATTTGAAATGTCGGCTTTTCAGAAA GGGCATGAGCAGGAAAACCATCACGAACTAGTTGATATTGGGAAA AAGATTGTTGAAAAATGTTATAACAATCCACTTGCTATAACGGTG GTAGGAAGTCTTCTTTATGGAGAGGAGATAAGTAAGTGGCGGTCA TTTGAAATGAGTGAGTTGGCCAAAATTGGCAATGGGGATAATAAG ATTTTGCCGATATTAAAGCTCAGTTACCATAATCTTATACCCTCGT TGAAGAGTTGCTTCAGTTATTGTGCAGTGTTTCCCAAGGATCATGA AATAAAGAAGGAGATGTTGATTGATCTTTGGATAGCACAAGGATA CGTTGTGGCACTTGATGGAGGTCAAAGTATAGAAGATGCTGCCGA AGAACATTTTGTAATTTTGTTACGGAGATGTTTCTTTCAAGATGTA AAGAAGGATGAATATGGTGATGTTGATTCTGTTAAAATCCACGAC TTGATGCACGATGTCGCCCAAGAAGTGGGGAGGGAGGAAATATGT GTAGTGAATGATAATACAAAGAACTTGGGTGATAAAATCCGTCAT GTACATGGTGATGTCAATAGATATGCACAAAGAGTCTCTCTGTGT AGCCATAGCCATAAGATTCGTTCGTATATTGGTGGTGATTGTGAAA AACGTTGTGTGGATACACTAATAGACAAGTGGATGTGTCTTAGGA TGTTGGACTTGTCATGGTCGGATGTTAAAAATTTGCCTAATTCAAT AGGTAAATTGTTGCACTTGAGGTATCTTAACCTGTCAGATAATAGA AATCTAAAGATACTTCCTGATGCAATTACAAGACTGCATAATTTGC AGACACTGCTTTTAAAAGATTGCAGAAGTTTAAAGGAGTTGCCAA AAGATTTTTGCAAATTGGTCAAACTGAGACACTTGGATTTATGGG GTTGTGATGATTTGATTGGTATGCCATTTGGAATGGATAAGCTAAC TAGTCTTAGAATACTACCAAACATTGTGGTGGGTAGGAAGGAACA AAGTGTTGATGATGAGCTGAAAGCCCTTAAAGGCCTCACCGAGAT AAAAGGCGACATTGATATCAAAATCTGTGAAAATTATAGAATAGT TGAAGGCATGAATGACACAGGAGGAGCTGGGTATTTGAAGAGCAT GAAACATCTCAGGGAGATTGGTATTACATTTGATGGTGGATGTGTT AACCCTGAAGCTGTGTTGGCAACCCTAGAGCCACCTTCAAATATC AAGAGCTTATCTATACATCGTTTTGATGGTAAAACACTTCCAGTAT |
| | GGGGAAGAGCAGAGATTAATTGGGCAATCTCCCTCTCACATCTTG TCGACATCCAGCTTTGGCATTGTCGTAATTTGCAGGAGATGCCAGT GCTGAGTAAACTGCCTCATTTGAAATCACTGGAACTTTATAATTTG ATTAGTTTAGAGTACATGGAGAGCACAAGCAGAAGCAGTAGCAGT GACACAGAAGCAGCAACACCAGAATTACCAACATTCTTCCCTTCC CTTGAAAAACTTACACTTTGGGGTCTGGAAAAGTTGAAGGGTTTG GGGAACAGGAGATCGAGTAGTTTTCCCCGCCTCTCTGAATTGAAA ATCATGGAATGCCCAGATCTAACGTGGTTTCCTCCCTGTCCAAGCC TTGAAAAACTTACACTTTGGCGTCTGGACAAGTTGAAGGGTTTGG GAACCGGAGATCGAGTACTTTTCCCCGCCTCTCTGAATTGGAAATC AAGAAATGCCCAGATCTAACGTCATTTCCTTCTTGTCCAAGCCTTG AGAAGTTGGAATTGAAAGAAAGCAATGAAGCATTGCAAATAATA GTAAAAATAACAACAAGAGGTAAAGAAAAAGAAGAGAACAATAA TGCTGGTGTTAGAAATTCACAAGATGATGACAAAGTCAAATTACG GAAGATGGTGATAGACAATCTGGGTTATCTCAAATCACTGCCCAC AAATTGTCTTACTCACCTCGACCTTACAATAAGTGATTCCAAGGAG GGGGAGGGTGAATGGAAGTTGGGGATGCATTTCAGAAGTGTGTA TCTTCTTTGAGAAGCCTCACCATAATCGGAAATCACGGAATAAAT AAAGTGAAGAGACTGTCTGGAAGAACAGGGTTGGAGCATTTCACT CTGTTGGAATCACTCAAACTTTCAGATATAGAAGACCAGGAAGAT GAGGGCGAAGACAACATCATATTCTGGAAATCCTTTCCTCAAAAC CTCCGCAGTTTGAGAATTAAAGACTCTGACAAAATGACAAGTTTG CCCATGGGGATGCAGTACTTAACCTCCCTCCAAACCCTCTATCTAC ACCATTGTTATGAATTGAATTCCCTTCCAGAATGGATAAGCAGCTT ATCATCTCTTCAATACCTGCGCATATACTACTGTCCAGCCCTGAAA TCACTACCAGAAGCAATGCGGAACCTCACCTCCCTTCAGACACTT GGGATATCGGATTGTCCAGACCTAGTTAAAAGATGCAGAAAACCC AACGGCAAGGACTATCCCAAAATTCAACACATCCCCTATTGGAGT ATAGAACATCAGGTTATAACTAGCTTGTAACTAACTTGTAACTACC TAGTATAAATACAGTAGTTTGTACTATTTTACATTCAATTACACAA TTAATAAAATGTAGACTCTCACTCTCTCTCTCTAAGCCACGAGCTC CAAGCTCGTCAATGGCTTCCCTTCTCTGTTCTTGCTTTCTTCTTTCC TCTTCAATTCACAAATTCAACATGGTATCAGAGCGGGACGATCCTT GCTCTTCACTTCCGCACAAAATTTTCGTTCAATTCAACCCATCAAA TTTTTTTTTCCCCCAAATTTTCTCGAATTCGGTCAAAATTCGACGA ATTAGGGATTCAATTTACCCTGATTTCTTCTGATTCCATTCAATGAT TGTTCATTTCGAATCTTGAATCAAATAATTGTTGATTCTGGATTCC CCAAATTCTAGGGTTCTTGAAGGATTTACAAGAATCTGGCATTGCT GATAGATTCTTGAAGCAATTTGCGTCTCCGTGTTCCTCGGTGGTCT TGAGTTTGTTTCCGTATTCGCTGCTCTCATCTTTACTGGGGATTGTG GTCTGATTTCTTGGCTTCCTCTGTCGATGATGTGATTGGTAATACTT AAAACCCCTCTCTCTCTCTTTCCGAAATTATTGATGCTGGTTCGTCATT TTTTTTTTTGGAATCATCTCAGTTTATCGCCGCAATTTGAGTTGTTG TTGGGTAATTGTTGTTGCTGCCGATGATGTTTTGTGAATTTGAGAA TTGTTAGAATGATTCTTGTTCAATCAATTTGGTTCTCATACTCTAAT GGAAGCCTGTTTTGGAGCGACGAATTATGCAATTCTGAGATTTCTT TTGATCCTTATTTCTTTTCTTCACTTGAATTTCTGGTGTTTGTGAGT AATTCTTGGTTAATGTTTGATCTGGGTAGTTCTTGGGTTTACTGAA GACGTTTCTTGAAGGTTTTGACAGAAAAGCTGAGGTTTAATTCCAA AATTCTTCTGTCCAATTACATTTTTATTGTTGATGGTTCTTATGTGA GAACTAGACTGAGTTTTTTTATGAAATTGTTTCGACCTTCAGATG GATTCGAGAGATTTGAGTTCATTTCTTTGATGAATGTGTTAGAAA AGGTTTTGGTGCAGTGACCATTTTAAACCAAATAGAGTTACATAA ATATTGGGATTCTTTTCTGGGAATGTAGTTAGGAGTTGAAATCTTT |
| | TGGAGCTGCTTTACCATAAAACCCAGCCTCAGAGTCTGTTAACCA GTTAGGACCGTGTAAACATGATCCCAGGCTGCATTTGCGTTATCAG ATTTGATTCAGTTTTGGAATTGTGGATTTTGAGGGTTTAAAAGCTT ACAGTTGCTCCTGGAGAATGGTGTGAGCAATATAGGAATTCAGCA CTAGTATTGCAGAAAATGAAGCTTGGTTGTTGATTGTTGGCATGTT TTGTTGCCATTGTTTTGGGTTGATGTTTTCCTTTTCTTTTGAATGTT GGCACGATTCAACATTTCTTTCCTGCAACAGATTTGGAGTTCAGTA CCTGTATAATCAGGTCAATTTTGTTCATTTTTCCCAGCAACAGATC TGGAGAATCAGAACCTGTAAAANNNNNNNNNNNNNNNNNNNNNN NNNNNNNNNNNNNNNNNNNNNNNNNNNNNNNNNNNNNNNACCCA AAGAGGTCAGTTTTCATTGATCCATTGTGATCATTCTTTTGATGAG ACCCATTGAGGCTCATTTCTTCAAGGCAATATTGGAAGTTGTAGAT TGATATGAGCAGTTGGTACAACAGCAACAAAAGTGGCCAGCATCT ATGCTTGTTCATGAGGAGTTCTTGGTGCAGAGTTAATGAAGAGTCT GTTTTGAAGCTTTCAAACTGAAGATGTTTATCACCATCTCCAGTTT GAGGGGGGGTATTGGAGTATAGAACATCAGGTTATAACTAGCTTG TAACTAACTTGTAACTACCTAGTATAAATACAGTAGTTTGTACNNN NNNNNNNNNNNNNNNNNNNNNNNNNNNNNNNNNNNNNNNNNNN NNNNCCACGAGCTCCAAGCTCGTCAATGGCTTCCCTTCTCTGTTCT TGCTTTCTTCTTTCCTCTTCAATTCACAAATTCAACATCCCCAAAAT TGTAAGTCATTGCAGAAAGTAATTTATTCATTTATATTTATTTTATG CTTAGAATGATATACGCAGTCGTCCTTTGGTTTCAAATCTTGAATT TGGTTTTTGTTTTCTTTCTTTGTTTCTTTATTCAACACCAGCCCATTT ATGATTGATTCATTAAAAAAAGGATGGAGTTTATGGATTTGAAG AAGACAACGAATTGAGATTCCTGGGGTTTCTTTTTGTTGGGGTTG GATTTCATGTATATGTTGCTGATTAAATACGAGACTGATGATGATG ATGTGTTTATGGGTTTTAAATCAGATTAAATATATGGGAAATGCAA GTTAATTTGGGATGCACATAAGGTGTTTGCTGAAATGTCTATGAGA AATGTTGTTTCTTGGACTTAGAATGATATACACTGTCGTCCTTTGG TTTCCAATCTTACATTTGGTTTGTGTTTTCTTAGTTTGTTTCTTTAAT CAACACCAACCCGTTTTTTTAAACTACCTGCAACTACTAATTTAC GTTTACCCTGTATCTCAGGTACTAAATGAATATTGGTGATTTTCAG TTACTCAACACTAGCTTGATCCTGAACGCACCCAACCTTCAGGTTA GAATCCGGCTTACTCATCCTTTTGTCCAGTTTTCAAGTAATTGTTTT GGCAGGATCAATTCTCTAATTGTTGTACACCGTATATTGCAATTTA TAGTGACTACAGTTAATGAATGTTTACAAAAAATTAGTCATGTAA AAACTTCTTCTCTGTCCATTACATAAACTCTTTTTCTCTTTCTAACT TATCATGTTCATGTCTAAACAATTAAACATGCTCACATCAATGTTC ATTTAAGCTAACTTACTTCTGTAAGAGAGCGAGCTAGTTAAAAAC TCCTTTAACTTTCTGTTTTATACTCAGGACATGGATTGATGCAAGC ATGAAGAACTTCGGGAATTTGCTAAAACTCTACCAAAGCGATGAG AGTTTGGACTTTATTTCACTTGAAGTCAGGGACTGTCAACAAAGCC ACAGTGTGCATGTTGGCTGTTTCACTTGGACGATAAAAAGGTTTAT TTAATTGTTTTCCTAAGTGTATTTGGCTTACAAGCTTTTACTTTTCA CTTGAAAGGGTTTTTCTTGTTTTAAGCTTTTCGAATTAGAGTTTTCG GTTGAAGTAAGAGTAGTCGTATTAGTCTTTTACCTAAGGAAGACTC TTTTTTGTAATTTTCAGACTATGCAATTCAAGTTTTCGAGTGTTTTC TTGCTTGTGTGATTGTGAGTTGGTGAATTCGTCTTTCATACATTTTG AGATTATCAGAAGCTTTATGCTCCACCGGTAGTCTAGTACCTTTTC TGTTACTGTGCAGGGAAGTAATCTGGTACCTTCTATATATATGGAA AAACATACATTATACATTATGCAAAATTCTTACAGGTTAGTTACTT CCTGGAACTTCATTTACACTTAGTTTTTTTGTTCCATTCCCTCGGA ATCAAGTCATTCCCTCTGAGAAATATGTAATGAACTTCTGTATGTT GCTGTTTGGTTCCTGTTTTAATCTTCAATTTTCTTGTATAGTTACAG |
| | CTGCATTTACAATGAAGTTTAAGCAGACACTCTCTTTATATAGTGC CTCTTTCTGGAGCACCGTAGAGCTGTCTGTGGTTGATCACCATCTG CTGCCGAGAGATTCAGCAATCGCGTGTTTGATCAGGTAAAAGTTTT TATGTCAATGTGTTTTTTTTCCGTTTGATCAATTTATGTCTGTATT CAGATTCTTATCTTCTTACAGTAGCATAACACATTGTTTCTTTCATT TATGTAAACTGTTTCAAGATTACAGAGATGTATGCTTCAGTCGACA TTGATGATAACTTAAGATGGCATTCCTACAACAGTTGCAGGCGCA TTCTAACTCCGGCAATTCTAGTTAGGCAAGAGGAGCATTGCCAAT ACCTGCCACCTCTGGGATTTACTATACCAGGGTTGAAGTTATGGA AGACACCAGCTATGCACAAGCCTTCAAGGGGTCATCCTACATAAC AAGTTGAACCAACCAATTGCTTGTTGGTTCAGTGGTAATTGAAGCT GAATTTGGTAGGGATGGCCCGTGTTCGATCCCCACAACAACAATT GGGAGGGGACTGGAACCTATCCACACAGAACTCGCCCTGAATCCG GATTAGCCCTAAGGGTGAACGGGGTGCTAACACCAAAAAAAAAA ACATAACAAGTTGAACCAAACATACTTTGTTTGAATTGAAGATTTA GTGATTTCATTTGATCGATTGAGATGTCTTATTATAAGCGTATATG CTCTTGGATTTGGCCACTTAGGTGTTGTTTGACAATTGGACATTAA CTCGCTTTTATATTTTCTTTTCTCTTAGGAAAGGTGATCCTGAGAAT TTATATTGGAACACTTTTTTTTTCTCACTAGCTTTAAAAAGTGTTC TGTGTTACCTGCAATTCAATTTGATTATTTTCACATAGTTTTACCT GAAAAAGTGTTACCTGAAAAAGTGTTACCTGAAAATCAACTGACA TAAGTTTTTGTTTGGATCCAATTAAGGACACTAGATAAATCGGAAT AAATAATCAACCAATTAAGTACTTCATAATTAAATATGAAGTGTA TTATTATCTTATGCTTGTGACATTGAAGGATGTTATGATATTTTAA CTCAATACCTTGCAAAATATACTGG |
| SEQ ID No:2: cds alpha-WOLF 8 | ATGGCCGAAATCGGATACTCGGTTTGTGCGAAACTCATCGAAGTG ATTGGCAGTGAGCTGATCAAAGAGATTTGTGACACATGGGGTTAC AAATCTCTTCTTGAGGACCTCAACAAAACTGTATTGACGGTCAGG AACGTTCTCATTCAAGCCGGGGTGATGCGGGAGCTTACTAGTGAA CAACAAGGTTTCATTGCAGACCTTAAAGATGTTGTTTATGATGCTG ATGACTTGTTCGACAAGTTACTCACTCGTGCTGAGCGAAAACAGA TTGATGGAAACGAAATCTCTGAAAAGGTACGTCGTTTCTTTTCCTC TAGTAACAAGATCGGTCAAGCTTACTACATGTCTCGTAAGGTTAA GGAAATTAAGAAGCAGTTGGATGAAATTGTTGATAGGCATACAAA ATTTGGGTTTAGTGCCGAGTTTATACCTGTTTGTAGGGAAAGGGGG AACGAGAGGGAAACACGTTCATATATAGATGTCAAGAATATTCTT GGGAGGGATAAAGATAAGAATGATATCATAGATAGGTTGCTTAAT CGTAATGGTAATGAAGCTTGTAGTTTCCTGACCATAGTGGGAGCG GGAGGATTGGGAAAAACTGCTCTTGCACAACTTGTGTTCAATGAT GAAAGGGTCAAAATTGAGTTCCATGATTTGAGGTATTGGGTTTGT GTCTCTGATCAAGATGGGGGCCAATTTGATGTGAAAGAAATCCTT TGTAAGATTTTAGAGGTGGTTACTAAGGAGAAAGTTGATAATAGT TCCACATTGGAATTGGTACAAAGCCAATTTCAAGAGAAGTTAAGA GGAAAGAAGTACTTCCTTGTTCTTGATGATGTATGGAACGAAGAT CGTGAGAAGTGGCTTCCTTTGGAAGAGTTGTTAATGTTGGGTCAA GGGGGAAGCAAGGTTGTAGTGACCGCACGTTCAGAGAAGACAGC AAATGTCATAGGGAAAAGACATTTTTATACACTGGAATGTTTGTC ACCAGATTATTCATGGAGCTTATTTGAAATGTCGGCTTTTCAGAAA GGGCATGAGCAGGAAAACCATCACGAACTAGTTGATATTGGGAAA AAGATTGTTGAAAAATGTTATAACAATCCACTTGCTATAACGGTG GTAGGAAGTCTTCTTTATGGAGAGGAGATAAGTAAGTGGCGGTCA TTTGAAATGAGTGAGTTGGCCAAAATTGGCAATGGGGATAATAAG ATTTTGCCGATATTAAAGCTCAGTTACCATAATCTTATACCCTCGT TGAAGAGTTGCTTCAGTTATTGTGCAGTGTTTCCCAAGGATCATGA |
| | AATAAAGAAGGAGATGTTGATTGATCTTTGGATAGCACAAGGATA CGTTGTGGCACTTGATGGAGGTCAAAGTATAGAAGATGCTGCCGA AGAACATTTTGTAATTTTGTTACGGAGATGTTTCTTTCAAGATGTA AAGAAGGATGAATATGGTGATGTTGATTCTGTTAAAATCCACGAC TTGATGCACGATGTCGCCCAAGAAGTGGGGAGGGAGGAAATATGT GTAGTGAATGATAATACAAAGAACTTGGGTGATAAAATCCGTCAT GTACATGGTGATGTCAATAGATATGCACAAAGAGTCTCTCTGTGT AGCCATAGCCATAAGATTCGTTCGTATATTGGTGGTGATTGTGAAA AACGTTGTGTGGATACACTAATAGACAAGTGGATGTGTCTTAGGA TGTTGGACTTGTCATGGTCGGATGTTAAAAATTTGCCTAATTCAAT AGGTAAATTGTTGCACTTGAGGTATCTTAACCTGTCAGATAATAGA AATCTAAAGATACTTCCTGATGCAATTACAAGACTGCATAATTTGC AGACACTGCTTTTAAAAGATTGCAGAAGTTTAAAGGAGTTGCCAA AAGATTTTTGCAAATTGGTCAAACTGAGACACTTGGATTTATGGG GTTGTGATGATTTGATTGGTATGCCATTTGGAATGGATAAGCTAAC TAGTCTTAGAATACTACCAAACATTGTGGTGGGTAGGAAGGAACA AAGTGTTGATGATGAGCTGAAAGCCCTTAAAGGCCTCACCGAGAT AAAAGGCGACATTGATATCAAAATCTGTGAAAATTATAGAATAGT TGAAGGCATGAATGACACAGGAGGAGCTGGGTATTTGAAGAGCAT GAAACATCTCAGGGAGATTGGTATTACATTTGATGGTGGATGTGTT AACCCTGAAGCTGTGTTGGCAACCCTAGAGCCACCTTCAAATATC AAGAGCTTATCTATACATCGTTTTGATGGTAAAACACTTCCAGTAT GGGGAAGAGCAGAGATTAATTGGGCAATCTCCCTCTCACATCTTG TCGACATCCAGCTTTGGCATTGTCGTAATTTGCAGGAGATGCCAGT GCTGAGTAAACTGCCTCATTTGAAATCACTGGAACTTTATAATTTG ATTAGTTTAGAGTACATGGAGAGCACAAGCAGAAGCAGTAGCAGT GACACAGAAGCAGCAACACCAGAATTACCAACATTCTTCCCTTCC CTTGAAAAACTTACACTTTGGGGTCTGGAAAAGTTGAAGGGTTTG GGGAACAGGAGATCGAGTAGTTTTCCCCGCCTCTCTGAATTGAAA ATCATGGAATGCCCAGATCTAACGTGGTTTCCTCCCTGTCCAAGCC TTGAAAAACTTACACTTTGGCGTCTGGACAAGTTGAAGGGTTTTGG GAACCGGAGATCGAGTACTTTTCCCCGCCTCTCTGAATTGGAAATC AAGAAATGCCCAGATCTAACGTCATTTCCTTCTTGTCCAAGCCTTG AGAAGTTGGAATTGAAAGAAAGCAATGAAGCATTGCAAATAATA GTAAAAATAACAACAAGAGGTAAAGAAAAAGAAGAGAACAATAA TGCTGGTGTTAGAAATTCACAAGATGATGACAAAGTCAAATTACG GAAGATGGTGATAGACAATCTGGGTTATCTCAAATCACTGCCCAC AAATTGTCTTACTCACCTCGACCTTACAATAAGTGATTCCAAGGAG GGGGAGGGTGAATGGGAAGTTGGGGATGCATTTCAGAAGTGTGTA TCTTCTTTGAGAAGCCTCACCATAATCGGAAATCACGGAATAAAT AAAGTGAAGAGACTGTCTGGAAGAACAGGGTTGGAGCATTTCACT CTGTTGGAATCACTCAAACTTTCAGATATAGAAGACCAGGAAGAT GAGGGCGAAGACAACATCATATTCTGGAAATCCTTTCCTCAAAAC CTCCGCAGTTTGAGAATTAAAGACTCTGACAAAATGACAAGTTTG CCCATGGGGATGCAGTACTTAACCTCCCTCCAAACCCTCTATCTAC ACCATTGTTATGAATTGAATTCCCTTCCAGAATGGATAAGCAGCTT ATCATCTCTTCAATACCTGCGCATATACTACTGTCCAGCCCTGAAA TCACTACCAGAAGCAATGCGGAACCTCACCTCCCTTCAGACACTT GGGATATCGGATTGTCCAGACCTAGTTAAAAGATGCAGAAAACCC AACGGCAAGGACTATCCCAAAATTCAACACATCCCCTATTGGAGT ATAGAACATCAGGTTATAACTAGCTTGTAA |
| SEQ ID No:3: cds of alpha- | ATGGCCGAAATCGGATACTCGGTTTGTGCGAAACTCATCGAAGTG ATTGGCAGTGAGCTGATCAAAGAGATTTGTGACACATGGGGTTAC AAATCTCTTCTTGAGGACCTCAACAAAACTGTATTGACGGTCAGG |
| WOLF 8 (isoform 1) | AACGTTCTCATTCAAGCCGGGGTGATGCGGAGCTTACTAGTGAA CAACAAGGTTTCATTGCAGACCTTAAAGATGTTGTTTATGATGCTG ATGACTTGTTCGACAAGTTACTCACTCGTGCTGAGCGAAAACAGA TTGATGGAAACGAAATCTCTGAAAAGGTACGTCGTTTCTTTTCCTC TAGTAACAAGATCGGTCAAGCTTACTACATGTCTCGTAAGGTTAA GGAAATTAAGAAGCAGTTGGATGAAATTGTTGATAGGCATACAAA ATTTGGGTTTAGTGCCGAGTTTATACCTGTTTGTAGGGAAAGGGGG AACGAGAGGGAAACACGTTCATATATAGATGTCAAGAATATTCTT GGGAGGGATAAAGATAAGAATGATATCATAGATAGGTTGCTTAAT CGTAATGGTAATGAAGCTTGTAGTTTCCTGACCATAGTGGGAGCG GGAGGATTGGGAAAAACTGCTCTTGCACAACTTGTGTTCAATGAT GAAAGGGTCAAAATTGAGTTCCATGATTTGAGGTATTGGGTTTGT GTCTCTGATCAAGATGGGGGCCAATTTGATGTGAAAGAAATCCTT TGTAAGATTTTAGAGGTGGTTACTAAGGAGAAAGTTGATAATAGT TCCACATTGGAATTGGTACAAAGCCAATTTCAAGAGAAGTTAAGA GGAAAGAAGTACTTCCTTGTTCTTGATGATGTATGGAACGAAGAT CGTGAGAAGTGGCTTCCTTTGGAAGAGTTGTTAATGTTGGGTCAA GGGGGAAGCAAGGTTGTAGTGACCGCACGTTCAGAGAAGACAGC AAATGTCATAGGGAAAAGACATTTTTATACACTGGAATGTTTGTC ACCAGATTATTCATGGAGCTTATTTGAAATGTCGGCTTTTCAGAAA GGGCATGAGCAGGAAAACCATCACGAACTAGTTGATATTGGGAAA AAGATTGTTGAAAAATGTTATAACAATCCACTTGCTATAACGGTG GTAGGAAGTCTTCTTTATGGAGAGGAGATAAGTAAGTGGCGGTCA TTTGAAATGAGTGAGTTGGCCAAAATTGGCAATGGGGATAATAAG ATTTTGCCGATATTAAAGCTCAGTTACCATAATCTTATACCCTCGT TGAAGAGTTGCTTCAGTTATTGTGCAGTGTTTCCCAAGGATCATGA AATAAAGAAGGAGATGTTGATTGATCTTTGGATAGCACAAGGATA CGTTGTGGCACTTGATGGAGGTCAAAGTATAGAAGATGCTGCCGA AGAACATTTTGTAATTTTGTTACGGAGATGTTTCTTTCAAGATGTA AAGAAGGATGAATATGGTGATGTTGATTCTGTTAAAATCCACGAC TTGATGCACGATGTCGCCCAAGAAGTGGGGAGGGAGGAAATATGT GTAGTGAATGATAATACAAAGAACTTGGGTGATAAAATCCGTCAT GTACATGGTGATGTCAATAGATATGCACAAAGAGTCTCTCTGTGT AGCCATAGCCATAAGATTCGTTCGTATATTGGTGGTGATTGTGAAA AACGTTGTGTGGATACACTAATAGACAAGTGGATGTGTCTTAGGA TGTTGGACTTGTCATGGTCGGATGTTAAAAATTTGCCTAATTCAAT AGGTAAATTGTTGCACTTGAGGTATCTTAACCTGTCAGATAATAGA AATCTAAAGATACTTCCTGATGCAATTACAAGACTGCATAATTTGC AGACACTGCTTTTAAAAGATTGCAGAAGTTTAAAGGAGTTGCCAA AAGATTTTTGCAAATTGGTCAAACTGAGACACTTGGATTTATGGG GTTGTGATGATTTGATTGGTATGCCATTTGGAATGGATAAGCTAAC TAGTCTTAGAATACTACCAAACATTGTGGTGGGTAGGAAGGAACA AAGTGTTGATGATGAGCTGAAAGCCCTTAAAGGCCTCACCGAGAT AAAAGGCGACATTGATATCAAAATCTGTGAAAATTATAGAATAGT TGAAGGCATGAATGACACAGGAGGAGCTGGGTATTTGAAGAGCAT GAAACATCTCAGGGAGATTGGTATTACATTTGATGGTGGATGTGTT AACCCTGAAGCTGTGTTGGCAACCCTAGAGCCACCTTCAAATATC AAGAGCTTATCTATACATCGTTTTGATGGTAAAACACTTCCAGTAT GGGGAAGAGCAGAGATTAATTGGGCAATCTCCCTCTCACATCTTG TCGACATCCAGCTTTGGCATTGTCGTAATTTGCAGGAGATGCCAGT GCTGAGTAAACTGCCTCATTTGAAATCACTGGAACTTTATAATTTG ATTAGTTTAGAGTACATGGAGAGCACAAGCAGAAGCAGTAGCAGT GACACAGAAGCAGCAACACCAGAATTACCAACATTCTTCCCTTCC CTTGAAAAACTTACACTTTGGGGTCTGGAAAAGTTGAAGGGTTTG |
| | GGGAACAGGAGATCGAGTAGTTTTCCCCGCCTCTCTGAATTGAAA ATCATGGAATGCCCAGATCTAACGTGGTTTCCTCCCTGTCCAAGCC TTGAAAAACTTACACTTTGGCGTCTGGACAAGTTGAAGGGTTTTGG GAACCGGAGATCGAGTACTTTTCCCCGCCTCTCTGAATTGGAAATC AAGAAATGCCCAGATCTAACGTCATTTCCTTCTTGTCCAAGCCTTG AGAAGTTGGAATTGAAAGAAAGCAATGAAGCATTGCAAATAATA GTAAAAATAACAACAAGAGGTAAAGAAAAAGAAGAGAACAATAA TGCTGGTGTTAGAAATTCACAAGATGATGACAAAGTCAAATTACG GAAGATGGTGATAGACAATCTGGGTTATCTCAAATCACTGCCCAC AAATTGTCTTACTCACCTCGACCTTACAATAAGTGATTCCAAGGAG GGGGAGGGTGAATGGGAAGTTGGGGATGCATTTCAGAAGTGTGTA TCTTCTTTGAGAAGCCTCACCATAATCGGAAATCACGGAATAAAT AAAGTGAAGAGACTGTCTGGAAGAACAGGGTTGGAGCATTTCACT CTGTTGGAATCACTCAAACTTTCAGATATAGAAGACCAGGAAGAT GAGGGCGAAGACAACATCATATTCTGGAAATCCTTTCCTCAAAAC CTCCGCAGTTTGAGAATTAAAGACTCTGACAAAATGACAAGTTTG CCCATGGGGATGCAGTACTTAACCTCCCTCCAAACCCTCTATCTAC ACCATTGTTATGAATTGAATTCCCTTCCAGAATGGATAAGCAGCTT ATCATCTCTTCAATACCTGCGCATATACTACTGTCCAGCCCTGAAA TCACTACCAGAAGCAATGCGGAACCTCACCTCCCTTCAGACACTT GGGATATCGGATTGTCCAGACCTAGTTAAAAGATGCAGAAAACCC AACGGCAAGGACTATCCCAAAATTCAACACATCCCCTATTGGAGT ATAGAACATCAGGTACTAAATGAATATTGGTGA |
| SEQ ID No:4: cds of alpha-WOLF 8 (isoform 2) | ATGGCCGAAATCGGATACTCGGTTTGTGCGAAACTCATCGAAGTG ATTGGCAGTGAGCTGATCAAAGAGATTTGTGACACATGGGGTTAC AAATCTCTTCTTGAGGACCTCAACAAAACTGTATTGACGGTCAGG AACGTTCTCATTCAAGCCGGGGTGATGCGGGAGCTTACTAGTGAA CAACAAGGTTTCATTGCAGACCTTAAAGATGTTGTTTATGATGCTG ATGACTTGTTCGACAAGTTACTCACTCGTGCTGAGCGAAAACAGA TTGATGGAAACGAAATCTCTGAAAAGGTACGTCGTTTCTTTTCCTC TAGTAACAAGATCGGTCAAGCTTACTACATGTCTCGTAAGGTTAA GGAAATTAAGAAGCAGTTGGATGAAATTGTTGATAGGCATACAAA ATTTGGGTTTAGTGCCGAGTTTATACCTGTTTGTAGGGAAAGGGGG AACGAGAGGGAAACACGTTCATATATAGATGTCAAGAATATTCTT GGGAGGGATAAAGATAAGAATGATATCATAGATAGGTTGCTTAAT CGTAATGGTAATGAAGCTTGTAGTTTCCTGACCATAGTGGGAGCG GGAGGATTGGGAAAAACTGCTCTTGCACAACTTGTGTTCAATGAT GAAAGGGTCAAAATTGAGTTCCATGATTTGAGGTATTGGGTTTGT GTCTCTGATCAAGATGGGGGCCAATTTGATGTGAAAGAAATCCTT TGTAAGATTTTAGAGGTGGTTACTAAGGAGAAAGTTGATAATAGT TCCACATTGGAATTGGTACAAAGCCAATTTCAAGAGAAGTTAAGA GGAAAGAAGTACTTCCTTGTTCTTGATGATGTATGGAACGAAGAT CGTGAGAAGTGGCTTCCTTTGGAAGAGTTGTTAATGTTGGGTCAA GGGGGAAGCAAGGTTGTAGTGACCGCACGTTCAGAGAAGACAGC AAATGTCATAGGGAAAAGACATTTTTATACACTGGAATGTTTGTC ACCAGATTATTCATGGAGCTTATTTGAAATGTCGGCTTTTCAGAAA GGGCATGAGCAGGAAAACCATCACGAACTAGTTGATATTGGGAAA AAGATTGTTGAAAAATGTTATAACAATCCACTTGCTATAACGGTG GTAGGAAGTCTTCTTTATGGAGAGGAGATAAGTAAGTGGCGGTCA TTTGAAATGAGTGAGTTGGCCAAAATTGGCAATGGGGATAATAAG ATTTTGCCGATATTAAAGCTCAGTTACCATAATCTTATACCCTCGT TGAAGAGTTGCTTCAGTTATTGTGCAGTGTTTCCCAAGGATCATGA AATAAAGAAGGAGATGTTGATTGATCTTTGGATAGCACAAGGATA CGTTGTGGCACTTGATGGAGGTCAAAGTATAGAAGATGCTGCCGA AGAACATTTTGTAATTTTGTTACGGAGATGTTTCTTTCAAGATGTA AAGAAGGATGAATATGGTGATGTTGATTCTGTTAAAATCCACGAC TTGATGCACGATGTCGCCCAAGAAGTGGGGAGGGAGGAAATATGT GTAGTGAATGATAATACAAAGAACTTGGGTGATAAAATCCGTCAT GTACATGGTGATGTCAATAGATATGCACAAAGAGTCTCTCTGTGT AGCCATAGCCATAAGATTCGTTCGTATATTGGTGGTGATTGTGAAA AACGTTGTGTGGATACACTAATAGACAAGTGGATGTGTCTTAGGA TGTTGGACTTGTCATGGTCGGATGTTAAAAATTTGCCTAATTCAAT AGGTAAATTGTTGCACTTGAGGTATCTTAACCTGTCAGATAATAGA AATCTAAAGATACTTCCTGATGCAATTACAAGACTGCATAATTTGC AGACACTGCTTTTAAAAGATTGCAGAAGTTTAAAGGAGTTGCCAA AAGATTTTTGCAAATTGGTCAAACTGAGACACTTGGATTTATGGG GTTGTGATGATTTGATTGGTATGCCATTTGGAATGGATAAGCTAAC TAGTCTTAGAATACTACCAAACATTGTGGTGGGTAGGAAGGAACA AAGTGTTGATGATGAGCTGAAAGCCCTTAAAGGCCTCACCGAGAT AAAAGGCGACATTGATATCAAAATCTGTGAAAATTATAGAATAGT TGAAGGCATGAATGACACAGGAGGAGCTGGGTATTTGAAGAGCAT GAAACATCTCAGGGAGATTGGTATTACATTTGATGGTGGATGTGTT AACCCTGAAGCTGTGTTGGCAACCCTAGAGCCACCTTCAAATATC AAGAGCTTATCTATACATCGTTTTGATGGTAAAACACTTCCAGTAT GGGGAAGAGCAGAGATTAATTGGGCAATCTCCCTCTCACATCTTG TCGACATCCAGCTTTGGCATTGTCGTAATTTGCAGGAGATGCCAGT GCTGAGTAAACTGCCTCATTTGAAATCACTGGAACTTTATAATTTG |
| | ATTAGTTTAGAGTACATGGAGAGCACAAGCAGAAGCAGTAGCAGT GACACAGAAGCAGCAACACCAGAATTACCAACATTCTTCCCTTCC CTTGAAAAACTTACACTTTGGGGTCTGGAAAAGTTGAAGGGTTTG GGGAACAGGAGATCGAGTAGTTTTCCCCGCCTCTCTGAATTGAAA ATCATGGAATGCCCAGATCTAACGTGGTTTCCTCCCTGTCCAAGCC TTGAAAAACTTACACTTTGGCGTCTGGACAAGTTGAAGGGTTTTGG GAACCGGAGATCGAGTACTTTTCCCCGCCTCTCTGAATTGGAAATC AAGAAATGCCCAGATCTAACGTCATTTCCTTCTTGTCCAAGCCTTG AGAAGTTGGAATTGAAAGAAAGCAATGAAGCATTGCAAATAATA GTAAAAATAACAACAAGAGGTAAAGAAAAAGAAGAGAACAATAA TGCTGGTGTTAGAAATTCACAAGATGATGACAAAGTCAAATTACG GAAGATGGTGATAGACAATCTGGGTTATCTCAAATCACTGCCCAC AAATTGTCTTACTCACCTCGACCTTACAATAAGTGATTCCAAGGAG GGGGAGGGTGAATGGGAAGTTGGGGATGCATTTCAGAAGTGTGTA TCTTCTTTGAGAAGCCTCACCATAATCGGAAATCACGGAATAAAT AAAGTGAAGAGACTGTCTGGAAGAACAGGGTTGGAGCATTTCACT CTGTTGGAATCACTCAAACTTTCAGATATAGAAGACCAGGAAGAT GAGGGCGAAGACAACATCATATTCTGGAAATCCTTTCCTCAAAAC CTCCGCAGTTTGAGAATTAAAGACTCTGACAAAATGACAAGTTTG CCCATGGGGATGCAGTACTTAACCTCCCTCCAAACCCTCTATCTAC ACCATTGTTATGAATTGAATTCCCTTCCAGAATGGATAAGCAGCTT ATCATCTCTTCAATACCTGCGCATATACTACTGTCCAGCCCTGAAA TCACTACCAGAAGCAATGCGGAACCTCACCTCCCTTCAGACACTT GGGATATCGGATTGTCCAGACCTAGTTAAAAGATGCAGAAAACCC AACGGCAAGGACTATCCCAAAATTCAACACATCCCCTATTGGAGT ATAGAACATCAGTTACTCAACACTAGCTTGATCCTGAACGCACCC AACCTTCAGGACATGGATTGA |
| SEQ ID No:5: protein sequence of alpha-WOLF 8 | MAEIGYSVCAKLIEVIGSELIKEICDTWGYKSLLEDLNKTVLTVRNVLI QAGVMRELTSEQQGFIADLKDVVYDADDLFDKLLTRAERKQIDGNEI SEKVRRFFSSSNKIGQAYYMSRKVKEIKKQLDEIVDRHTKFGFSAEFIP VCRERGNERETRSYIDVKNILGRDKDKNDIIDRLLNRNGNEACSFLTI VGAGGLGKTALAQLVFNDERVKIEFHDLRYWVCVSDQDGGQFDVK EILCKILEVVTKEKVDNSSTLELVQSQFQEKLRGKKYFLVLDDVWNE DREKWLPLEELLMLGQGGSKVVVTARSEKTANVIGKRHFYTLECLSP DYSWSLFEMSAFQKGHEQENHHELVDIGKKIVEKCYNNPLAITVVGS LLYGEEISKWRSFEMSELAKIGNGDNKILPILKLSYHNLIPSLKSCFSYC AVFPKDHEIKKEMLIDLWIAQGYVVALDGGQSIEDAAEEHFVILLRRC FFQDVKKDEYGDVDSVKIHDLMHDVAQEVGREEICVVNDNTKNLGD KIRHVHGDVNRYAQRVSLCSHSHKIRSYIGGDCEKRCVDTLIDKWMC LRMLDLSWSDVKNLPNSIGKLLHLRYLNLSDNRNLKILPDAITRLHNL QTLLLKDCRSLKELPKDFCKLVKLRHLDLWGCDDLIGMPFGMDKLT SLRILPNIVVGRKEQSVDDELKALKGLTEIKGDIDIKICENYRIVEGMN DTGGAGYLKSMKHLREIGITFDGGCVNPEAVLATLEPPSNIKSLSIHRF DGKTLPVWGRAEINWAISLSHLVDIQLWHCRNLQEMPVLSKLPHLKS LELYNLISLEYMESTSRSSSSDTEAATPELPTFFPSLEKLTLWGLEKLK GLGNRRSSSFPRLSELKIMECPDLTWFPPCPSLEKLTLWRLDKLKGFG NRRSSTFPRLSELEIKKCPDLTSFPSCPSLEKLELKESNEALQIIVKITTR GKEKEENNNAGVRNSQDDDKVKLRKMVIDNLGYLKSLPTNCLTHLD LTISDSKEGEGEWEVGDAFQKCVSSLRSLTIIGNHGINKVKRLSGRTG LEHFTLLESLKLSDIEDQEDEGEDNIIFWKSFPQNLRSLRIKDSDKMTS LPMGMQYLTSLQTLYLHHCYELNSLPEWISSLSSLQYLRIYYCPALKS LPEAMRNLTSLQTLGISDCPDLVKRCRKPNGKDYPKIQHIPYWSIEHQ VITSL |
| SEQ ID No:6: protein sequence of alpha-WOLF 8 (isoform 1) | MAEIGYSVCAKLIEVIGSELIKEICDTWGYKSLLEDLNKTVLTVRNVLI QAGVMRELTSEQQGFIADLKDVVYDADDLFDKLLTRAERKQIDGNEI SEKVRRFFSSSNKIGQAYYMSRKVKEIKKQLDEIVDRHTKFGFSAEFIP VCRERGNERETRSYIDVKNILGRDKDKNDIIDRLLNRNGNEACSFLTI VGAGGLGKTALAQLVFNDERVKIEFHDLRYWVCVSDQDGGQFDVK EILCKILEVVTKEKVDNSSTLELVQSQFQEKLRGKKYFLVLDDVWNE DREKWLPLEELLMLGQGGSKVVVTARSEKTANVIGKRHFYTLECLSP DYSWSLFEMSAFQKGHEQENHHELVDIGKKIVEKCYNNPLAITVVGS LLYGEEISKWRSFEMSELAKIGNGDNKILPILKLSYHNLIPSLKSCFSYC AVFPKDHEIKKEMLIDLWIAQGYVVALDGGQSIEDAAEEHFVILLRRC FFQDVKKDEYGDVDSVKIHDLMHDVAQEVGREEICVVNDNTKNLGD KIRHVHGDVNRYAQRVSLCSHSHKIRSYIGGDCEKRCVDTLIDKWMC LRMLDLSWSDVKNLPNSIGKLLHLRYLNLSDNRNLKILPDAITRLHNL QTLLLKDCRSLKELPKDFCKLVKLRHLDLWGCDDLIGMPFGMDKLT SLRILPNIVVGRKEQSVDDELKALKGLTEIKGDIDIKICENYRIVEGMN DTGGAGYLKSMKHLREIGITFDGGCVNPEAVLATLEPPSNIKSLSIHRF DGKTLPVWGRAEINWAISLSHLVDIQLWHCRNLQEMPVLSKLPHLKS LELYNLISLEYMESTSRSSSSDTEAATPELPTFFPSLEKLTLWGLEKLK GLGNRRSSSFPRLSELKIMECPDLTWFPPCPSLEKLTLWRLDKLKGFG NRRSSTFPRLSELEIKKCPDLTSFPSCPSLEKLELKESNEALQIIVKITTR GKEKEENNNAGVRNSQDDDKVKLRKMVIDNLGYLKSLPTNCLTHLD LTISDSKEGEGEWEVGDAFQKCVSSLRSLTIIGNHGINKVKRLSGRTG LEHFTLLESLKLSDIEDQEDEGEDNIIFWKSFPQNLRSLRIKDSDKMTS LPMGMQYLTSLQTLYLHHCYELNSLPEWISSLSSLQYLRIYYCPALKS LPEAMRNLTSLQTLGISDCPDLVKRCRKPNGKDYPKIQHIPYWSIEHQ VLNEYW |
| SEQ ID No:7: protein sequence of alpha-WOLF 8 (isoform 2) | MAEIGYSVCAKLIEVIGSELIKEICDTWGYKSLLEDLNKTVLTVRNVLI QAGVMRELTSEQQGFIADLKDVVYDADDLFDKLLTRAERKQIDGNEI SEKVRRFFSSSNKIGQAYYMSRKVKEIKKQLDEIVDRHTKFGFSAEFIP VCRERGNERETRSYIDVKNILGRDKDKNDIIDRLLNRNGNEACSFLTI VGAGGLGKTALAQLVFNDERVKIEFHDLRYWVCVSDQDGGQFDVK EILCKILEVVTKEKVDNSSTLELVQSQFQEKLRGKKYFLVLDDVWNE DREKWLPLEELLMLGQGGSKVVVTARSEKTANVIGKRHFYTLECLSP DYSWSLFEMSAFQKGHEQENHHELVDIGKKIVEKCYNNPLAITVVGS LLYGEEISKWRSFEMSELAKIGNGDNKILPILKLSYHNLIPSLKSCFSYC AVFPKDHEIKKEMLIDLWIAQGYVVALDGGQSIEDAAEEHFVILLRRC FFQDVKKDEYGDVDSVKIHDLMHDVAQEVGREEICVVNDNTKNLGD KIRHVHGDVNRYAQRVSLCSHSHKIRSYIGGDCEKRCVDTLIDKWMC LRMLDLSWSDVKNLPNSIGKLLHLRYLNLSDNRNLKILPDAITRLHNL QTLLLKDCRSLKELPKDFCKLVKLRHLDLWGCDDLIGMPFGMDKLT SLRILPNIVVGRKEQSVDDELKALKGLTEIKGDIDIKICENYRIVEGMN DTGGAGYLKSMKHLREIGITFDGGCVNPEAVLATLEPPSNIKSLSIHRF DGKTLPVWGRAEINWAISLSHLVDIQLWHCRNLQEMPVLSKLPHLKS LELYNLISLEYMESTSRSSSSDTEAATPELPTFFPSLEKLTLWGLEKLK GLGNRRSSSFPRLSELKIMECPDLTWFPPCPSLEKLTLWRLDKLKGFG NRRSSTFPRLSELEIKKCPDLTSFPSCPSLEKLELKESNEALQIIVKITTR GKEKEENNNAGVRNSQDDDKVKLRKMVIDNLGYLKSLPTNCLTHLD LTISDSKEGEGEWEVGDAFQKCVSSLRSLTIIGNHGINKVKRLSGRTG LEHFTLLESLKLSDIEDQEDEGEDNIIFWKSFPQNLRSLRIKDSDKMTS LPMGMQYLTSLQTLYLHHCYELNSLPEWISSLSSLQYLRIYYCPALKS LPEAMRNLTSLQTLGISDCPDLVKRCRKPNGKDYPKIQHIPYWSIEHQ LLNTSLILNAPNLQDMD |
| SEQ ID No:8: Forward primer LRR domain (Alpha) | ACAAGTGGATGTGTCTTAGG |
| SEQ ID No:9: Reverse primer LRR domain | TTCGCCCTCATCTTCCTGG |
| SEQ ID No:10: Forward primer LRR domain (Beta) | TCACGTGGGTTGTGTTGT |
| SEQ ID No:11: Amplicon of LRR domain of the alpha-WOLF 8 allele | ACAAGTGGATGTGTCTTAGGATGTTGGACTTGTCATGGTCGGATGT TAAAAATTTGCCTAATTCAATAGGTAAATTGTTGCACTTGAGGTAT CTTAACCTGTCAGATAATAGAAATCTAAAGATACTTCCTGATGCA ATTACAAGACTGCATAATTTGCAGACACTGCTTTTAAAAGATTGCA GAAGTTTAAAGGAGTTGCCAAAAGATTTTTGCAAATTGGTCAAAC TGAGACACTTGGATTTATGGGGTTGTGATGATTTGATTGGTATGCC ATTTGGAATGGATAAGCTAACTAGTCTTAGAATACTACCAAACAT TGTGGTGGGTAGGAAGGAACAAAGTGTTGATGATGAGCTGAAAGC CCTTAAAGGCCTCACCGAGATAAAAGGCGACATTGATATCAAAAT CTGTGAAAATTATAGAATAGTTGAAGGCATGAATGACACAGGAGG AGCTGGGTATTTGAAGAGCATGAAACATCTCAGGGAGATTGGTAT TACATTTGATGGTGGATGTGTTAACCCTGAAGCTGTGTTGGCAACC CTAGAGCCACCTTCAAATATCAAGAGCTTATCTATACATCGTTTTG ATGGTAAAACACTTCCAGTATGGGGAAGAGCAGAGATTAATTGGG CAATCTCCCTCTCACATCTTGTCGACATCCAGCTTTGGCATTGTCG TAATTTGCAGGAGATGCCAGTGCTGAGTAAACTGCCTCATTTGAA ATCACTGGAACTTTATAATTTGATTAGTTTAGAGTACATGGAGAGC ACAAGCAGAAGCAGTAGCAGTGACACAGAAGCAGCAACACCAGA ATTACCAACATTCTTCCCTTCCCTTGAAAAACTTACACTTTGGGGT CTGGAAAAGTTGAAGGGTTTGGGGAACAGGAGATCGAGTAGTTTT CCCCGCCTCTCTGAATTGAAAATCATGGAATGCCCAGATCTAACGT GGTTTCCTCCCTGTCCAAGCCTTGAAAAACTTACACTTTGGCGTCT GGACAAGTTGAAGGGTTTTGGGAACCGGAGATCGAGTACTTTTCC CCGCCTCTCTGAATTGGAAATCAAGAAATGCCCAGATCTAACGTC ATTTCCTTCTTGTCCAAGCCTTGAGAAGTTGGAATTGAAAGAAAGC AATGAAGCATTGCAAATAATAGTAAAAATAACAACAAGAGGTAA AGAAAAAGAAGAGAACAATAATGCTGGTGTTAGAAATTCACAAG ATGATGACAAAGTCAAATTACGGAAGATGGTGATAGACAATCTGG GTTATCTCAAATCACTGCCCACAAATTGTCTTACTCACCTCGACCT TACAATAAGTGATTCCAAGGAGGGGGAGGGTGAATGGGAAGTTG GGGATGCATTTCAGAAGTGTGTATCTTCTTTGAGAAGCCTCACCAT AATCGGAAATCACGGAATAAATAAAGTGAAGAGACTGTCTGGAA GAACAGGGTTGGAGCATTTCACTCTGTTGGAATCACTCAAACTTTC AGATATAGAAGACCAGGAAGATGAGGGCGAA |
| SEQ ID No:12: amino acid | KWMCLRMLDLSWSDVKNLPNSIGKLLHLRYLNLSDNRNLKILPDAIT RLHNLQTLLLKDCRSLKELPKDFCKLVKLRHLDLWGCDDLIGMPFG MDKLTSLRILPNIVVGRKEQSVDDELKALKGLTEIKGDIDIKICENYRI |
| sequence encoded by amplicon of LRR domain of alpha Wolf 8 | VEGMNDTGGAGYLKSMKHLREIGITFDGGCVNPEAVLATLEPPSNIK SLSIHRFDGKTLPVWGRAEINWAISLSHLVDIQLWHCRNLQEMPVLS KLPHLKSLELYNLISLEYMESTSRSSSSDTEAATPELPTFFPSLEKLTLW GLEKLKGLGNRRSSSFPRLSELKIMECPDLTWFPPCPSLEKLTLWRLD KLKGFGNRRSSTFPRLSELEIKKCPDLTSFPSCPSLEKLELKESNEALQI IVKITTRGKEKEENNNAGVRNSQDDDKVKLRKMVIDNLGYLKSLPTN CLTHLDLTISDSKEGEGEWEVGDAFQKCVSSLRSLTIIGNHGINKVKR LSGRTGLEHFTLLESLKLSDIEDQEDEGE |
| SEQ ID No:13: Amplicon of LRR domain of the beta-WOLF 0 allele | TCACGTGGGTTGTGTTGTCGATAGAGATCCAGAAATAGTCTTTTA TGTAGCAATAAGATTCGTTCGTATATTAGCGGTCGCTGCATAAAG AATCCGGTGGATTCACAAATAGACAACTGGATGTGCCTTAGGGTG TTGGACTTGTCAGATTCATGTGTTAAAGATTTGTCTGATTCAATAG GTAAGCTGCTGCACTTAAGGTATCTTAACCTCTCTTCTAATATAAA GTTGGAGATAATCCCTGATGCAATTACAAGACTGCATAACTTGCA GACACTACTTTTAGAAGATTGCAGAAGTTTAAAGGAGTTGCCAAA AGATTTTTGCAAATTGGTCAAACTGAGGCACTTGGAATTACAGGG TTGTCATGATTTGATTGGTATGTCATTTGGAATGGATAAGCTAACT AGTCTTAGAATACTACCAAACATTGTGGTGGGTAGGAAGGAACAA AGTGTTGATGATGAGCTGAAAGCCCTAAAAGGCCTCACCGAGATA AAAGGCTCCATTGATATCACAATCTATTCAAAATATAGAAGAGTT GAAGGCATGAATGGCACAGGAGGAGGAGCTGGGTATTTGAAGAG CATGAAACATCTCACGGGGGTTAATATTACATTTGATGAAGGTGG ATGTGTTAACCCTGAAGCTGTGTATTTGAAGAGCATGAAACATCTC ACGAGGGTTATTATTATATTTGATTATAAAGGTGGATGTGTTAACC CTGAAGCTGTGTTGGCAACCCTAGAGCCACCTTCAAATATCAAGA GGTTAGAGATGTGGCATTACAGTGGTACAACAATTCCAGTATGGG GAAGAGCAGAGATTAATTGGGCAATCTCCCTCTCACATCTTGTCG ACATCACGCTTGAAGATTGTTACAATTTGCAGGAGATGCCAGTGC TGAGTAAACTGCCTCATTTGAAATCACTGGAACTTACAGAGTTGG ATAACTTAGAGTACATGGAGAGTAGAAGCAGCAGCAGTAGCAGT GACACAGAAGCAGCAACACCAGAATTACCAACATTCTTCCCTTCC CTTGAAAAACTTACACTTTGGCGTCTGGACAAGTTGAAGGGTTTG GGAACAGGAGATCGAGTAGTTTTCCCCGCCTCTCTAAATTGGAAA TCTGGAAATGTCCAGATCTAACGTCATTTCCTTCTTGTCCAAGCCT TGAAGAGTTGGAATTGAAAGAAAACAATGAAGCGTTGCAAATAAT AGTAAAAATAACAACAACAAGAGGTAAAGAAGAAAAAGAAGAA GACAAGAATGCTGGTGTTGGAAATTCACAAGATGATGACAATGTC AAATTATGGAAGGTGGAAATAGACAATCTGGGTTATCTCAAATCA CTGCCCACAAATTGTCTGACTCACCTCGACCTTACAATAAGTGATT CCAAGGAGGGGGAGGGTGAATGGGAAGTTGGGGATGCATTTCAG AAGTGTGTATCTTCTTTGAGAAGCCTCACCATAATCGGAAATCACG GAATAAATAAAGTGAAGAGACTGTCTGGAAGAACAGGGTTGGAG CATTTCACTCTGTTGGAATCACTCAAACTTTCAGATATAGAAGACC AGGAAGATGAGGGCGAA |
| SEQ ID No:14: amino acid sequence encoded by amplicon of LRR | HVGCVVDRDPEIVFLCSNKIRSYISGRCIKNPVDSQIDNWMCLRVLDL SDSCVKDLSDSIGKLLHLRYLNLSSNIKLEIIPDAITRLHNLQTLLLEDC RSLKELPKDFCKLVKLRHLELQGCHDLIGMSFGMDKLTSLRILPNIVV GRKEQSVDDELKALKGLTEIKGSIDITIYSKYRRVEGMNGTGGGAGY LKSMKHLTGVNITFDEGGCVNPEAVYLKSMKHLTRVIIIFDYKGGCV NPEAVLATLEPPSNIKRLEMWHYSGTTIPVWGRAEINWAISLSHLVDI TLEDCYNLQEMPVLSKLPHLKSLELTELDNLEYMESRSSSSSSDTEAA TPELPTFFPSLEKLTLWRLDKLKGFGNRRSSSFPRLSKLEIWKCPDLTS FPSCPSLEELELKENNEALQIIVKITTTRGKEEKEEDKNAGVGNSQDD |
| domain Beta Wolf 0 (Viroflay) | DNVKLWKVEIDNLGYLKSLPTNCLTHLDLTISDSKEGEGEWEVGDAF QKCVSSLRSLTIIGNHGINKVKRLSGRTGLEHFTLLESLKLSDIEDQED EGE |

The present invention will be further clarified in the Examples that follow and that are given for illustration purposes only and are not intended to limit the invention in any way.

### EXAMPLES

### EXAMPLE 1

### Testing for resistance to Peronospora farinosa f. sp. spinaciae in spinach plants

The resistance to downy mildew infection was assayed as described by Irish et al. (2008; Phytopathol. 98: 894-900), using a differential set. Spinach plants of the invention were sown along with spinach plants from different other genotypes (see **Table 3)** in trays containing Scotts Redi-Earth medium, and fertilized twice a week after seedling emergence with Osmocote Peter's (13-13-13) fertilizer (Scotts). Plants were inoculated with a sporangial suspension (2.5 × 10⁵/ml) of a pathogenic race of *Peronospora farinosa* f. sp. *spinaciae* at the first true leaf stage. In this manner, 16 officially recognized pathogenic races were tested.

The inoculated plants were placed in a dew chamber at 18°C with 100% relative humidity for a 24 h period, and then moved to a growth chamber at 18°C with a 12 h photoperiod for 6 days. After 6 days, the plants were returned to the dew chamber for 24 h to induce sporulation, and they were scored for disease reaction.

Plants for this specific test were scored as resistant, intermediately resistant, or susceptible based on symptoms of chlorosis and signs of pathogen sporulation on the cotyledons and true leaves, as described by Irish et al. (2007; Plant Dis. 91: 1392-1396). Plants exhibiting no evidence of chlorosis and sporulation were in this specific test considered as resistant. Resistant plants were re-inoculated to assess whether plants initially scored as resistant had escaped infection, or whether they were truly resistant. Plants that showed only symptoms of chlorosis, or sporulation occurring only on the tips of the cotyledons were scored as intermediately resistant. Plants showing more than these symptoms of downy mildew infection were scored as being susceptible.

**Table 1** shows the resistance of a plant carrying the alpha-WOLF 8 allele to each one of these pathogenic races. **Table 3** shows the differential set of spinach downy mildew races and the resistance of various spinach varieties (hybrids) to each one of these pathogenic races. A susceptible reaction is scored as "+" (indicating a successful infection by the fungus, with sporulation occurring on the entire cotyledon), and resistance is depicted as "-" (absence of sporulation on the cotyledons). A weak resistance response is indicated as "(-)", which in practice means a slightly reduced level of infection (with only symptoms of chlorosis, or sporulation only occurring on the tips of the cotyledons in the differential seedling test).

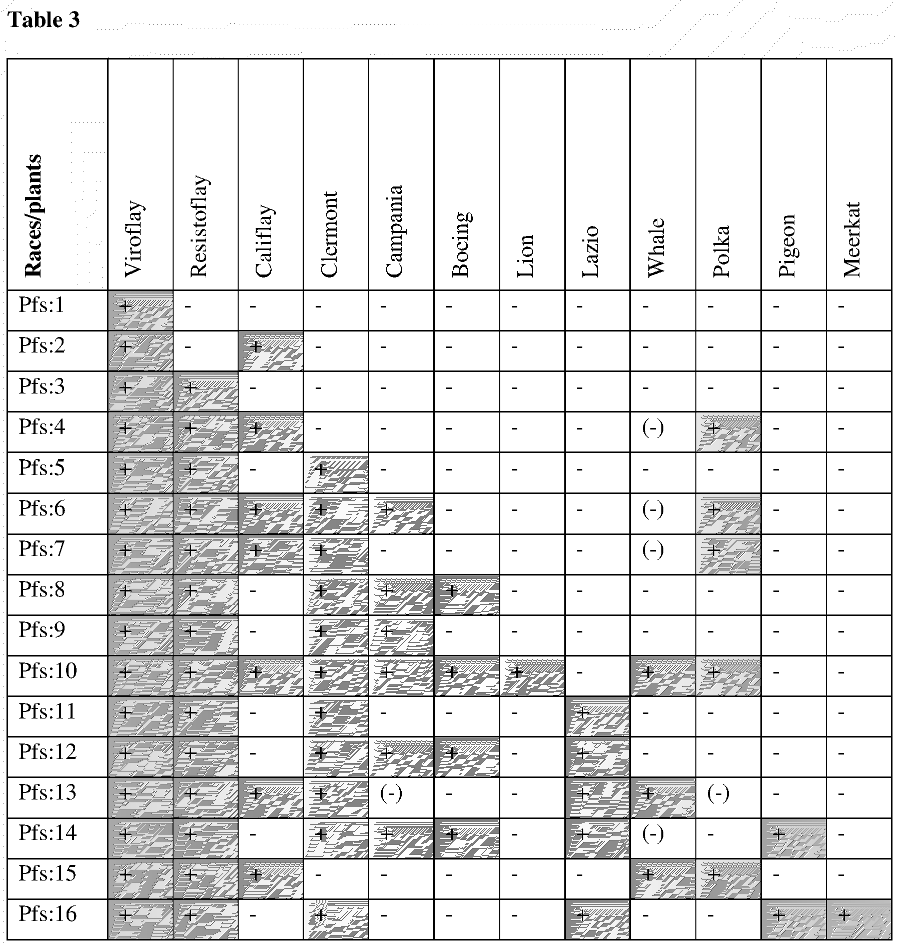

### EXAMPLE 2

### Amplification of the LRR domain-encoding region

The isolated genomic DNA of a spinach plant comprising the alpha-WOLF 8 allele, of which a representative sample of seed was deposited with the NCIMB under NCIMB accession number 42646 was used in polymerase chain reactions (PCR), using forward primer ACAAGTGGATGTGTCTTAGG (SEQ ID No:8) and reverse primer TTCGCCCTCATCTTCCTGG (SEQ ID No:9). The primer pair amplifies the LRR domain-encoding region of an alpha*-WOLF* gene, and has been designed for selectively amplifying part of a *WOLF* gene, and not of other CC-NBS-LRR protein-encoding genes.

PCR conditions for amplifying the LRR domain-encoding region of an alpha- *WOLF* gene using primers having SEQ ID No:8 and SEQ ID No:9 were as follows, using Platinum Taq enzyme (Thermo Fisher Scientific):
- 3 minutes at 95°C (initial denaturing step)
- 40 amplification cycles, each cycle consisting of: 30 seconds denaturation at 95°C, 30 seconds annealing at 60°C, and 30 seconds extension at 72°C
- 2 minutes at 72°C (final extension step)

The isolated genomic DNA of a spinach plant of variety Viroflay comprising the beta-WOLF 0 allele was used in polymerase chain reactions (PCR), using forward primer TCACGTGGGTTGTGTTGT (SEQ ID No:10) and reverse primer TTCGCCCTCATCTTCCTGG (SEQ ID No:9). The primer pair amplifies the LRR domain-encoding region of a beta-WOLF gene, and has been designed for selectively amplifying part of a *WOLF* gene, and not of other CC-NBS-LRR protein-encoding genes.

PCR conditions for amplifying the LRR domain-encoding region of a beta- *WOLF* gene using primers having SEQ ID No:9 and SEQ ID No:10 were as follows, using Platinum Taq enzyme (Thermo Fisher Scientific):
- 3 minutes at 95°C (initial denaturing step)
- 40 amplification cycles, each cycle consisting of: 30 seconds denaturation at 95°C, 50 seconds annealing at 58°C and 50 seconds extension at 72°C
- 2 minutes at 72°C (final extension step)

The PCR products were visualized on agarose gel (not shown), and DNA was purified from the PCR reaction. Subsequently the sequence of the PCR products was determined using methods well known in the art.

The sequence of the LRR domain of the alpha WOLF 8 allele amplified by primers having SEQ ID No:8 and SEQ ID No:9 is provided in **Table 2** under SEQ ID No:11.

The sequence of the LRR domain of the beta-WOLF 0 allele amplified by primers having SEQ ID No:9 and SEQ ID No:10 is provided in **Table 2** under SEQ ID No:13.

Finally, the obtained sequences were translated into the corresponding amino acid sequence of the LRR domain having SEQ ID No:12 and SEQ ID No:14 for the alpha-WOLF 8 allele and the beta-WOLF 0, respectively (See also **Table 2).**

If PCR products were to be sequenced using SMRT sequencing (Pacific Biosciences), PCR primers and PCR conditions were different.

To the above-mentioned forward primers the following standard amplification sequence was added: GCAGTCGAACATGTAGCTGACTCAGGTCAC.

To the reverse primer, the following standard amplification sequence was added: TGGATCACTTGTGCAAGCATCACATCGTAG.

### EXAMPLE 3

### Introducing alpha-WOLF 8 allele in a plant not carrying the allele

A spinach plant comprising the alpha-WOLF 8 allele, of which a representative sample of seed was deposited with the NCIMB under NCIMB accession number 42646 was crossed with a plant of variety Viroflay carrying the beta-WOLF 0 allele to obtain a F1 generation. Subsequently, a F1 plant was selfed to obtain a F2 population.

Plants of the F2 population were assayed as described in **Example 1** for resistance to *Peronospora farinosa* f. sp. *spinaciae* pfs:15. Approximately 75% of the plants scored completely resistant in the assay.

Genomic DNA of each plant of the same F2 population was isolated and used in two different polymerase chain reactions (PCR). The first PCR reaction was done using primers for amplifying the LRR domain of an alpha-WOLF allele and the second PCR reaction was done using primers for amplifying the LRR domain of a beta-WOLF allele, both as described in **Example 2.**

The PCR products were visualized on agarose gel (not shown), this demonstrated that approximately 25% of the plant only contained an alpha-WOLF fragment, approximately 50% contained both an alpha- and a beta-WOLF fragment, and that the remaining approximately 25% of the plants only contained a beta-WOLF fragment. The plants containing the alpha-WOLF fragment completely correlated with the plants that scored resistant for pfs:15. The plants only comprising the beta-WOLF fragment completely correlated with the plants that scored susceptible for pfs:15.

DNA from the PCR reaction was purified, and subsequently the sequence of the PCR products was determined. The alpha-WOLF PCR products gave a sequence that corresponded to the sequence of SEQ ID No:11, the genomic sequence of the LRR domain of the alpha-WOLF 8 allele. The beta-WOLF PCR products gave a sequence that corresponded to the sequence of SEQ ID No:13 the genomic sequence of the LRR domain of the beta-WOLF 0 allele.

## Claims

1. An allele designated alpha-WOLF 8 which confers resistance to at least one *Peronospora farinosa* f. sp. *spinaciae* race when present in a spinach plant, wherein the allele has a genomic nucleotide sequence of SEQ ID No:1, a coding sequence of SEQ ID No:2, SEQ ID No:3 or SEQ ID No:4, said allele encoding a CC-NBS-LRR protein that comprises in its amino acid sequence: a) the motif "MAEIGYSVC" at its N-terminus; and b) the motif "KWMCLR"; wherein the allele when homozygously present in a spinach plant confers complete resistance to *Peronospora farinosa* f. sp. *spinaciae* races pfs:1, pfs:2, pfs:6, pfs:8 and pfs:15, and confers intermediate resistance to pfs:5, pfs: 10 and pfs:16, and does not confer resistance to pfs:3, pfs:4, pfs:7, pfs:9, pfs: 11, pfs:12, pfs:13 and pfs:14.

2. A spinach plant comprising the allele as claimed in claim 1 , of which a representative sample of seed capable of growing into a plant comprising said allele was deposited with the NCIMB under NCIMB accession number 42646.

3. The spinach plant of claim 2, further comprising a genetic determinant resulting in resistance against *Peronospora farinosa* f. sp. *spinaciae* races pfs:1 to pfs:16.

4. Propagation material capable of developing into and/or being derived from a spinach plant as defined in claim 1, wherein the propagation material comprises the allele as claimed in claim 1 and wherein the propagation material is selected from a group consisting of a microspore, a pollen, an ovary, an ovule, an embryo, an embryo sac, an egg cell, a cutting, a root, a root tip, a hypocotyl, a cotyledon, a stem, a leaf, a flower, an anther, a seed, a meristematic cell, a protoplast, a cell, or a tissue culture thereof.

5. Cell of a spinach plant, which cell comprises the allele as claimed in claim 1 .

6. A hybrid spinach plant grown from seed produced by a method, comprising crossing a first parent spinach plant with a second parent spinach plant and harvesting the resultant hybrid spinach seed, wherein said first parent spinach plant and resultant hybrid spinach seed comprise the allele as claimed in claim 1 , wherein the first and/or second parent is optionally a plant of an inbred line.

7. Method for identifying or selecting a spinach plant carrying the allele as claimed in claim 1 , comprising determining the presence of a genomic nucleotide sequence or a part thereof in the genome of a plant, wherein said sequence has the sequence of SEQ ID No:1.

8. Method for identifying or selecting a spinach plant carrying the allele as claimed in claim 1, comprising determining the presence of a genomic nucleotide sequence or a part thereof in the genome of a plant, wherein said sequence has the sequence of SEQ ID No:2, SEQ ID No:3 or SEQ ID No:4, .

9. Method for identifying or selecting a spinach plant carrying the alpha-WOLF 8 allele as claimed in claim 1 which comprises determining the DNA sequence coding for the LRR domain, wherein said LRR domain has the sequence of SEQ ID No. 11..

10. The method of claim 9, wherein the LRR domain is determined by using a primer pair to amplify the LRR domain, wherein the forward primer is a nucleic acid molecule having the sequence of SEQ ID No:8 and wherein the reverse primer is a nucleic acid molecule having the sequence of SEQ ID No:9.

11. Primer pair comprising a forward primer which is a nucleic acid molecule having the sequence of SEQ ID No:8 and a reverse primer which is a nucleic acid molecule having the sequence of SEQ ID No:9.

## Patentansprüche

1. Als alpha-WOLF 8 bezeichnetes Allel, das Resistenz gegenüber mindestens eine *Peronospora farinosa* f. sp. *spinaciae*-Rasse verleiht, wenn es in einer Spinatpflanze vorhanden ist, wobei das Allel eine genomische Nukleotidsequenz der SEQ ID Nr. 1, eine kodierende Sequenz der SEQ ID Nr. 2, SEQ ID Nr. 3 oder SEQ ID Nr. 4 aufweist, wobei das Allel für ein CC-NBS-LRR-Protein kodiert, das in seiner Aminosäuresequenz umfasst: a) das Motiv "MAEIGYSVC" an seinem N-Terminus; und b) das Motiv "KWMCLR"; wobei das Allel, wenn es homozygot in einer Spinatpflanze vorhanden ist, vollständige Resistenz gegenüber *Peronospora farinosa* f. sp. *spinaciae*-Rassen pfs:1, pfs:2, pfs:6, pfs:8 und pfs:15 verleiht und intermediäre Resistenz gegenüber pfs:5, pfs:10 und pfs:16 verleiht und keine Resistenz gegenüber pfs:3, pfs:4, pfs:7, pfs:9, pfs:11, pfs:12, pfs:13 und pfs:14 verleiht.

2. Spinatpflanze, umfassend das Allel, wie in Anspruch 1 beansprucht, von der eine repräsentative Samenprobe, die in der Lage ist, zu einer Pflanze heranzuwachsen, die das Allel umfasst, bei der NCIMB unter der NCIMB-Zugangsnummer 42646 hinterlegt wurde.

3. Spinatpflanze nach Anspruch 2, ferner umfassend eine genetische Determinante, die zu einer Resistenz gegenüber *Peronospora farinosa* f. sp. *spinaciae*-Rassen pfs:1 bis pfs:16 führt.

4. Vermehrungsmaterial, das in der Lage ist, sich zu einer Spinatpflanze zu entwickeln und/oder von einer Spinatpflanze zu stammen, wie in Anspruch 1 definiert, wobei das Vermehrungsmaterial das Allel, wie in Anspruch 1 beansprucht, umfasst und wobei das Vermehrungsmaterial aus einer Gruppe ausgewählt ist, bestehend aus einer Mikrospore, einem Pollen, einem Fruchtknoten, einer Samenanlage, einem Keimling, einem Keimlingsbeutel, einer Eizelle, einem Steckling, einer Wurzel, einer Wurzelspitze, einem Hypokotyl, einem Keimblatt, einem Stängel, einem Blatt, einer Blüte, einem Staubbeutel, einem Samen, einer meristematischen Zelle, einem Protoplasten, einer Zelle oder einer Gewebekultur davon.

5. Zelle einer Spinatpflanze, wobei die Zelle das Allel nach Anspruch 1 umfasst.

6. Hybrid-Spinatpflanze, gezogen aus Samen, der durch ein Verfahren erzeugt wurde, umfassend das Kreuzen einer ersten Eltern-Spinatpflanze mit einer zweiten Eltern-Spinatpflanze und das Ernten des resultierenden Hybrid-Spinatsamens, wobei die erste Eltern-Spinatpflanze und der resultierende Hybrid-Spinatsamen das Allel, wie in Anspruch 1 beansprucht, umfassen, wobei der erste und/oder zweite Elternteil gegebenenfalls eine Pflanze einer Inzuchtlinie ist.

7. Verfahren zum Identifizieren oder Auswählen einer Spinatpflanze, die das Allel, wie in Anspruch 1 beansprucht, trägt, umfassend das Bestimmen des Vorhandenseins einer genomischen Nukleotidsequenz oder eines Teils davon in dem Genom einer Pflanze, wobei die Sequenz die Sequenz von SEQ ID Nr. 1 aufweist.

8. Verfahren zum Identifizieren oder Auswählen einer Spinatpflanze, die das Allel, wie in Anspruch 1 beansprucht, trägt, umfassend das Bestimmen des Vorhandenseins einer genomischen Nukleotidsequenz oder eines Teils davon in dem Genom einer Pflanze, wobei die Sequenz die Sequenz von SEQ ID Nr. 2, SEQ ID Nr. 3 oder SEQ ID Nr. 4 aufweist.

9. Verfahren zum Identifizieren oder Auswählen einer Spinatpflanze, die das alpha-WOLF 8-Allel trägt, wie in Anspruch 1 beansprucht, umfassend das Bestimmen der DNA-Sequenz, die für die LRR-Domäne kodiert, wobei die LRR-Domäne die Sequenz von SEQ ID Nr. 11 aufweist.

10. Verfahren nach Anspruch 9, wobei die LRR-Domäne durch Verwenden eines Primerpaars, um die LRR-Domäne zu verstärken, bestimmt wird, wobei der Vorwärtsprimer ein Nukleinsäuremolekül, das die Sequenz SEQ ID Nr. 8 aufweist, ist und/oder wobei der Rückwärtsprimer ein Nukleinsäuremolekül, das die Sequenz von SEQ ID Nr. 9 aufweist, ist.

11. Primerpaar, umfassend einen Vorwärtsprimer, der ein Nukleinsäuremolekül ist, das die Sequenz von SEQ ID Nr. 8 aufweist, und einen Rückwärtsprimer, der ein Nukleinsäuremolekül ist, das die Sequenz von SEQ ID Nr. 9 aufweist.

## Revendications

1. Allèle désigné alpha-WOLF 8 qui confère une résistance à au moins une race de *Peronospora farinosa* f sp. *spinaciae* lorsqu'il est présent dans un plant d'épinard, dans lequel l'allèle a une séquence nucléotidique génomique de SEQ ID NO:1, une séquence codante de SEQ ID NO:2, SEQ ID NO:3 ou SEQ ID NO:4, ledit allèle codant pour une protéine CC-NBS-LRR qui comprend dans sa séquence d'acides aminés : a) le motif « MAEIGYSVC » au niveau de son extrémité N-terminale ; et b) le motif « KWMCLR » ; dans lequel l'allèle, lorsqu'il est présent de manière homozygote dans un plant d'épinard, confère une résistance complète aux races de *Peronospora farinosa* f. sp. *spinaciae* pfs:1, pfs:2, pfs:6, pfs:8 et pfs:15, et confère une résistance intermédiaire à pfs:5, pfs:10 et pfs:16, et ne confère pas de résistance à pfs:3, pfs:4, pfs:7, pfs:9, pfs:11, pfs:12, pfs:13 et pfs:14.

2. Plant d'épinard comprenant l'allèle selon la revendication 1, dont un échantillon représentatif de semence capable de croître en un plant comprenant ledit allèle a été déposé auprès du NCIMB sous le numéro d'accès NCIMB 42646.

3. Plant d'épinard selon la revendication 2, comprenant en outre un déterminant génétique résultant en une résistance contre les races de *Peronospora farinosa* f. sp. *spinaciae* pfs:1 à pfs:16.

4. Matériau de propagation capable de se développer en un plant d'épinard selon la revendication 1 et/ou étant dérivé de celui-ci, dans lequel le matériau de propagation comprend l'allèle selon la revendication 1 et dans lequel le matériau de propagation est choisi dans un groupe constitué d'une microspore, d'un pollen, d'un ovaire, d'un ovule, d'un embryon, d'un sac embryonnaire, d'une cellule œuf, d'une bouture, d'une racine, d'un bout de racine, d'un hypocotyle, d'un cotylédon, d'une tige, d'une feuille, d'une fleur, d'une anthère, d'une graine, d'une cellule méristématique, d'un protoplaste, d'une cellule, ou d'une culture de tissu de ceux-ci.

5. Cellule d'un plant d'épinard, laquelle cellule comprend l'allèle selon la revendication 1.

6. Plant d'épinard hybride cultivé à partir de semence produite par un procédé, comprenant le croisement d'un premier plant d'épinard parent avec un second plant d'épinard parent et la récolte de la semence d'épinard hybride résultante, dans lequel ledit premier plant d'épinard parent et la semence d'épinard hybride résultante comprennent l'allèle selon la revendication 1, dans lequel le premier et/ou le second parent est facultativement un plant d'une lignée consanguine.

7. Procédé d'identification ou de sélection d'un plant d'épinard portant l'allèle selon la revendication 1, comprenant la détermination de la présence d'une séquence nucléotidique génomique ou d'une partie de celle-ci dans le génome d'un plant, dans lequel ladite séquence a la séquence de SEQ ID NO:1.

8. Procédé d'identification ou de sélection d'un plant d'épinard portant l'allèle selon la revendication 1, comprenant la détermination de la présence d'une séquence nucléotidique génomique ou d'une partie de celle-ci dans le génome d'un plant, dans lequel ladite séquence a la séquence de SEQ ID NO:2, SEQ ID NO:3 ou SEQ ID NO:4,.

9. Procédé d'identification ou de sélection d'un plant d'épinard portant l'allèle alpha-WOLF 8 selon la revendication 1, qui comprend la détermination de la séquence d'ADN codant pour le domaine LRR, dans lequel ledit domaine LRR a la séquence de SEQ ID NO. 11..

10. Procédé selon la revendication 9, dans lequel le domaine LRR est déterminé en utilisant une paire d'amorces pour amplifier le domaine LRR, dans lequel l'amorce sens est une molécule d'acide nucléique ayant la séquence de SEQ ID NO:8 et/ou dans lequel l'amorce antisens est une molécule d'acide nucléique ayant la séquence de SEQ ID NO:9.

11. Paire d'amorces comprenant une amorce sens qui est une molécule d'acide nucléique ayant la séquence de SEQ ID NO:8 et une amorce antisens qui est une molécule d'acide nucléique ayant la séquence de SEQ ID NO:9.
